# EUROPÄISCHE PATENTSCHRIFT

(11) **EP 2 881 132 B1**
(45) Veröffentlichungstag und Bekanntmachung des Hinweises auf die Patenterteilung: **18.10.2017**
(21) Anmeldenummer: 13195951.2
(22) Anmeldetag: 05.12.2013
(51) Int. Cl.: A61M 5/20, A61M 5/315

(54) **Injektionsvorrichtung mit einem Dosierglied und einer vorgespannten Ausschüttfeder**
Injection device with an improved metering member and a pre-tensioned ejection spring
Dispositif d'injection doté d'un organe de dosage et d'un ressort de versement précontraint

(43) Veröffentlichungstag der Anmeldung: 10.06.2015
(60) Teilanmeldung: 17172636.7
(73) Patentinhaber: TecPharma Licensing AG, 3401 Burgdorf (CH)
(72) Erfinder: Streit, Ursina, 3322 Schönbühl (CH); Schenker, Susanne, 4900 Langenthal (CH)
(74) Vertreter: Schwabe - Sandmair - Marx

(56) Entgegenhaltungen:
- EP-A1- 1 681 070
- EP-A1- 2 644 217
- WO-A1-2009/141005
- WO-A1-2013/144020

## Beschreibung

Die Erfindung betrifft eine Injektionsvorrichtung zum Verabreichen eines flüssigen Produkts, insbesondere eines Medikaments, wie z. B. Insulin zur Diabetestherapie. Im Besonderen betrifft die Erfindung eine Antriebs- und Dosiervorrichtung für eine solche Injektionsvorrichtung.

Der Begriff "Medikament" umfasst hier jede fließfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder coformulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe. Die Erfindung ist eine Weiterbildung der Erfindung aus der WO 2013/144021 A1. Aus der EP 2 644 217 A1 ist eine Injektionsvorrichtung gemäß dem Oberbegriff des Anspruchs 1 zur Verabreichung eines flüssigen Produkts bekannt, weiche zwei Druckfedern als Ausschüttfedern aufweist. Dabei ist die erste Druckfeder zwischen einem Widerlager und einem Zwischenglied und die zweite Druckfeder zwischen dem Zwischenlager und dem Vortriebsglied angebracht, wobei das Vortriebsglied mittels dem Widerlager verdrehfest mit dem Gehäuse gekoppelt ist. Es ist eine Aufgabe der Erfindung, eine Antriebs- und Dosiervorrichtung für eine Injektionsvorrichtung zur Verabreichung eines flüssigen Produkts anzugeben, die dem Verwender eine einfache Verwendung der Vorrichtung, insbesondere eine einfache Dosiseinstellung erlaubt und dennoch eine kompakte Bauweise ermöglicht.

Die Aufgabe wird durch die Merkmale des Anspruchs 1 gelöst. Vorteilhafte Weiterbildungen ergeben sich aus den abhängigen Ansprüchen, der Beschreibung und den Figuren.

Die Erfindung geht von einem Antriebsmechanismus für eine Injektionsvorrichtung zum Verabreichen eines flüssigen Medikaments oder Produkts aus. Der Antriebsmechanismus weist ein Gehäuse auf. Das Gehäuse ist vorzugsweise hülsenförmig und/oder länglich ausgebildet. Das Gehäuse kann sich z. B. entlang seiner Längsachse erstrecken.

Das Gehäuse kann optional einen Produktbehälter aufnehmen oder selbst den Produktbehälter bilden. Das Gehäuse kann ein- oder mehrteilig sein. Z. B. kann das Gehäuse einen proximalen Gehäuseteil bilden, der die Antriebs- und Dosiervorrichtung umfasst oder aufweist. Das Gehäuse kann ferner einen Produktbehälterhalter aufweisen, der den Produktbehälter, wie z. B. eine Karpule, aufnimmt und mit dem Gehäuse oder dem proximalen Gehäuseteil verbunden ist. Diese Verbindung kann derart sein, dass der Produktbehälterhalter und das Gehäuse oder der proximale Gehäuseteil nach dem Verbinden unlösbar, d. h. nur durch Zerstörung von Verbindungselementen lösbar ist. Eine solche Lösung ist insbesondere bei Einweginjektionsvorrichtungen von Vorteil, die nach dem das in dem Produktbehälter enthaltene Produkt vollständig ausgeschüttet ist, als Ganzes entsorgt werden. Alternativ kann der Produktbehälterhalter auch lösbar an dem Gehäuse befestigt werden, wodurch es möglich ist, wenngleich auch weniger bevorzugt, die Antriebs- und Dosiervorrichtung gegebenenfalls mehrfach zu verwenden, d. h. einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann z. B. eine Zeigeeinrichtung, insbesondere ein Fenster aufweisen, mittels der oder durch welches eine aktuell eingestellte Dosis, vorzugsweise von einer Skala eines Dosiseinstelleelements, ablesbar ist.

In einem ersten Aspekt, der zum Beispiel die Erfindung vorteilhaft weiterbildet, umfasst die Antriebs- und Dosiervorrichtung, die insbesondere zusammen mit dem Produktbehälter eine Injektionsvorrichtung bildet, neben einem Gehäuse ein Dosisanzeigeelement, über dessen Umfang eine Dosisskala angeordnet ist. Das Dosisanzeigeelement kann z. B. im Querschnitt ringförmig sein. Das Dosisanzeigeelement kann z. B. eine Dosisanzeigetrommel oder ein Dosisanzeigering sein. Die Dosisskala kann sich über den Umfang des Dosisanzeigeelements, vorzugsweise wendelförmig, erstrecken. Die Dosisskala umfasst vorzugsweise eine Vielzahl von Werten, die aneinandergereiht angeordnet sind und die Dosisskala ergeben. Vorzugsweise handelt es sich um Zahlenwerte, welche die gewünschte Produktdosis in internationalen Einheiten (I.U.) oder in Milligramm angeben.

Alternativ kann die Dosisskala ohne Steigung über den Umfang des Dosisanzeigeelements, wie z. B. des Dosisanzeigerings, angeordnet sein, wobei sich die Skalenwerte dann nach einer Umdrehung des Dosisanzeigeelements wiederholen. Bei einer Dosisskala mit Steigung, d. h. einer wendelförmigen Dosisskala kann das Dosisanzeigeelement, insbesondere die Dosisanzeigetrommel, mit mehr als einer Umdrehung gedreht werden, ohne dass sich die Skalenwerte wiederholen, wodurch vorteilhaft die Skalenwerte größer oder mehr Skalenwerte dargestellt werden können.

Die Antriebs- und Dosiervorrichtung umfasst ferner eine Zeigeeinrichtung, wobei das Dosisanzeigeelement zur Einstellung der zu verabreichenden Dosis relativ zu der Zeigeeinrichtung und insbesondere um eine Drehachse, die vorzugsweise der Längsachse der Antriebs- und Dosiervorrichtung oder/und des Dosisanzeigeelements entspricht, drehbar ist. Hierbei kann es sich um eine reine Drehbewegung, d. h. eine Drehbewegung ohne überlagerte Axialbewegung handeln. Vorzugsweise ist der Drehbewegung eine Axialbewegung überlagert, wodurch das Dosisanzeigeelement zur Einstellung der zu verabreichenden Dosis relativ zu der Zeigeeinrichtung schraubbar ist. Ein schraubbares Dosisanzeigeelement lässt sich vorteilhaft mit einer wendelförmigen Dosisskala kombinieren, wobei die Schraubbewegung und die Dosisskala vorteilhafterweise die gleiche Steigung aufweisen. Ein ohne Axialbewegung drehbares Dosisanzeigeelement lässt sich vorteilhaft mit einer steigungsfreien Dosisskala kombinieren.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann z. B. ein Fenster sein, das durch einen Durchbruch im Gehäuse oder durch einen transparenten Einsatz gebildet sein kann. Alternativ oder optional kann die Zeigeeinrichtung ein Pfeil sein oder einen Pfeil aufweisen, der z. B. zusätzlich zu dem Fenster den Wert der Dosisskala markiert, welcher der eingestellten Dosis entspricht. Dies ist z. B. dann von Vorteil, wenn in dem Fenster noch ein anderer Wert zumindest teilweise erscheint, um eine eindeutige Dosisauswahl sicherzustellen. Der Zeiger kann z. B. ein Vorsprung oder ein Aufdruck oder eine Kerbe oder dergleichen sein.

Die Antriebs- und Dosiervorrichtung umfasst ein Dosierglied, das z.B. als Dosierknopf ausgebildet ist und optional als Dosiseinstellglied bezeichnet werden kann. Das Dosierglied ist vorzugsweise vom Verwender (Patient, Arzt, medizinisches Hilfspersonal) der Antriebs- und Dosiervorrichtung greifbar und bildet vorzugsweise eine äußere, insbesondere von außen zugängliche Oberfläche der Antriebs- und Dosiervorrichtung. Zur Einstellung der auszuschüttenden oder zu verabreichenden Dosis wird das Dosierglied vorzugsweise vom Verwender ergriffen und relativ zu dem Gehäuse und insbesondere der Zeigeeinrichtung um eine Drehachse, die vorzugsweise der Längsachse der z.B. länglich ausgestalteten Antriebs- und Dosiervorrichtung entspricht, verdreht. Das Dosierglied ist vorzugsweise axialfest mit dem Gehäuse verbunden, insbesondere entlang einer Längsachse des Gehäuses verschiebefest, wodurch vorteilhaft die intuitive Handhabung der Vorrichtung durch den Verwender erleichtert wird, da er lediglich eine Drehbewegung des Dosierglieds zur Dosiseinstellung ausführen braucht.

Insbesondere kann das Dosisanzeigeelement zumindest während der Dosiseinstellung verdrehfest, aber z.B. axial verschiebbar mit dem Dosierglied verbunden oder gekoppelt sein. Für die intuitive Bedienung ist es vorteilhaft, wenn das Dosisanzeigeelement bei Drehung des Dosierglieds um einen Drehwinkel ebenfalls um diesen Drehwinkel verdreht wird.

Die Antriebs- und Dosiervorrichtung weist ein Betätigungsglied z.B. in der Gestalt eines Betätigungsknopfs auf. Das Betätigungsglied kann eine äußere Oberfläche der Antriebs- und Dosiervorrichtung bilden und/oder von außen zugänglich sein. Das Betätigungsglied kann am proximalen, insbesondere hinteren Ende der Antriebs- und Dosiervorrichtung gebildet sein oder dieses Ende bilden. Das Betätigungsglied lässt sich auf diese Weise vorteilhaft mit dem Daumen der Hand, die das Gehäuse umgreift betätigen, insbesondere drücken. Durch Loslassen des Betätigungsglieds kann die Betätigung beendet werden. Unter "betätigen" wird die Verschiebung des Betätigungsglieds in die Antriebs- und Dosiervorrichtung, insbesondere in distale Richtung verstanden, wodurch insbesondere eine Produktausschüttung bewirkt werden kann. Das Betätigungsglied ist vorteilhaft relativ zu dem Dosierglied verschiebbar und kann insbesondere von dem Dosierglied axial verschiebbar aufgenommen sein.

Das Betätigungsglied kann vorteilhaft gegen die Kraft einer Feder, insbesondere einer Rücksetz- oder Kupplungsfeder verschiebbar, insbesondere betätigbar sein, wodurch diese Feder gespannt wird. Durch Loslassen kann diese Feder das Betätigungsglied zurücksetzen, insbesondere relativ zu dem Dosierglied, insbesondere in proximale Richtung oder aus der Antriebs- und Dosiervorrichtung heraus, verschieben.

Die Antriebs- und Dosiervorrichtung umfasst ferner ein Lagerelement, mit dem das Dosisanzeigeelement in einem Eingriff ist. Dieser Eingriff bewirkt vorteilhaft die Dreh- oder Schraubbewegung des Dosisanzeigeelements relativ zu der Zeigeeinrichtung. Z. B. kann der Eingriff zwischen Dosisanzeigeelement und Lagerelement ein Gewindeeingriff sein.

Insbesondere kann das Lagerelement ein Außengewinde und das Dosisanzeigeelement ein Innengewinde aufweisen, wobei diese Gewinde ineinander greifen und dadurch bewirken, dass das Dosisanzeigeelement relativ zu dem Lagerelement schraubbar ist.

Insbesondere ist das Dosisanzeigeelement zwischen einer Maximaldosisposition und einer Nulldosisposition hin und her dreh- oder schraubbar. In der Nulldosisposition kann vorteilhaft die Dosis oder Ziffer "0" in der Zeigeeinrichtung ablesbar sein. In der Maximaldosisposition kann vorteilhaft die maximal mit der Antriebs- und Dosiervorrichtung ausschüttbare Produktdosis ablesbar sein.

In der Nulldosisposition kann das Dosisanzeigeelement gegen die Drehung in eine Drehrichtung gesperrt sein, nämlich in die Drehrichtung, die bewirken würde, dass eine Dosis kleiner als null eingestellt wird. In der Nulldosisposition kann das Dosisanzeigeelement vorzugsweise nur in die Drehrichtung bewegt werden, die eine Erhöhung der Dosis bewirkt. In der Maximaldosisposition ist das Dosisanzeigeelement vorzugsweise gegen die Drehung in eine Drehrichtung, nämlich in die Drehrichtung, die die Einstellung einer Dosis über die maximal einstellbare Dosis hinaus bewirken würde, blockiert. Das Dosisanzeigeelement kann in der Maximaldosisposition insbesondere nur in die Drehrichtung gedreht werden, die eine Verringerung der Produktdosis bewirkt.

Das Dosisanzeigeelement kann z. B. einen Anschlag aufweisen, der in der Nulldosisposition an einen Gegenanschlag anschlägt und somit die Drehung in eine Drehrichtung verhindert. Der gleiche oder ein zusätzlicher Anschlag des Dosisanzeigeelements kann die Drehung des Dosisanzeigeelements über die Maximaldosis hinaus verhindern. Insbesondere kann hierfür ein weiterer Gegenanschlag, nämlich ein Maximaldosisgegenanschlag vorgesehen sein. Dementsprechend kann der andere Gegenanschlag als Nulldosisgegenanschlag bezeichnet werden. Das Dosisanzeigeelement kann demnach einen Nulldosisanschlag für den Nulldosisgegenanschlag und einen Maximaldosisanschlag für den Maximaldosisgegenanschlag aufweisen. Vorzugsweise wirken der Anschlag oder die Anschläge in Umfangsrichtung und/oder in Axialrichtung.

Die Antriebs- und Dosiervorrichtung nach dem ersten Aspekt kann bevorzugt dergestalt sein, dass das Lagerelement zusammen mit dem Dosisanzeigeelement relativ zu dem Gehäuse und entlang der Drehachse, insbesondere in distale Richtung verschiebbar ist. Dieser Aspekt kann die Antriebs- und Dosiervorrichtung nach einem zweiten hierin beschriebenen Aspekt vorteilhaft weiterbilden. Alternativ kann das Dosisanzeigeelement ein Gewinde aufweisen, welches im Eingriff mit dem Gehäuse ist. Dadurch lässt sich das Dosisanzeigeelement zwar relativ zu dem Gehäuse hin und her schrauben, aber nicht unabhängig von der Schraubbewegung mit einer insbesondere reinen Axialbewegung verschieben.

Vorzugsweise ist das Betätigungsglied so mit dem Lagerelement gekoppelt, dass eine Verschiebung des Betätigungsglieds relativ zu dem Gehäuse und/oder dem Dosierglied eine Verschiebung des Lagerelements relativ zu dem Gehäuse und/oder dem Dosierglied insbesondere entlang der Längsachse der Antriebs- und Dosiervorrichtung bewirkt.

Dadurch dass das Dosisanzeigeelement erfindungsgemäß in einem Eingriff mit dem Lagerelement ist und sich das Lagerelement relativ zu dem Gehäuse und entlang der Drehachse verschieben lässt, lässt sich auch das Dosisanzeigeelement relativ zu dem Gehäuse und entlang der Drehachse unabhängig von der Dreh- oder Schraubbewegung, welche das Dosisanzeigeelement bei der Dosiseinstellung ausführt, verschieben. Die Antriebs- und Dosiervorrichtung nach dem zweiten Aspekt lässt sich im Grunde aber auch vorteilhaft mit dem alternativen Dosisanzeigeelement kombinieren, das in dem Gewindeeingriff mit dem Gehäuse oder einem gehäusefesten Element ist. In dieser Alternative kann das Lagerelement von dem Gehäuse gebildet sein oder Teil des Gehäuses sein, wobei das Lagerelement dann z. B. dreh- und axialfest in Bezug auf das übrige Gehäuse sein kann.

Vorteilhaft kann an der Zeigeeinrichtung und/oder an dem Dosisanzeigeelement abgelesen werden, dass das Lagerelement zusammen mit dem Dosisanzeigeelement verschoben wurde. Hierdurch lässt sich durch den Verwender kontrollieren, in welchem Betriebszustand sich die Antriebs- und Dosiervorrichtung befindet, d. h. ob die Antriebs- und Dosiervorrichtung insbesondere das Betätigungsglied für eine Ausschüttung betätigt oder unbetätigt ist.

In einer bevorzugten Variante kann das Betätigungsglied oder/und das Lagerelement zusammen mit dem Dosisanzeigeelement relativ zu der Zeigeeinrichtung, dem Gehäuse und entlang der Drehachse verschiebbar sein. Im Bereich der Zeigeeinrichtung, insbesondere in dem Fenster der Zeigeeinrichtung kann eine von der Dosisskala verschiedene Markierung erscheinen, wenn das Lagerelement verschoben ist. Die Markierung ist vorzugsweise an dem Dosisanzeigeelement angeordnet. Wenn das Lagerelement unverschoben ist, insbesondere die Antriebs- und Dosiervorrichtung für die Produktausschüttung unbetätigt ist, kann die Markierung außerhalb der Zeigeeinrichtung angeordnet sein, wie z. B. von einem Gehäuse oder einem anderen Element verdeckt sein. Wird das Lagerelement verschoben, insbesondere die Antriebs- und Dosiervorrichtung für die Produktausschüttung betätigt, kann die Markierung aus dem abgedeckten Bereich hervortreten, so dass sie insbesondere an oder in der Zeigeeinrichtung erscheint oder ablesbar ist. Wird die Betätigung der Antriebs- und Dosiervorrichtung unterbrochen oder beendet, kann das Lagerelement in die Ursprungsposition zurückkehren, wodurch die Markierung vorzugsweise aus dem Bereich der Zeigeeinrichtung entfernt und insbesondere verdeckt wird.

In einer alternativen Variante kann das Betätigungsglied oder/und das Lagerelement zusammen mit dem Dosisanzeigeelement und der Zeigeeinrichtung relativ zu dem Gehäuse und entlang der Drehachse verschiebbar sein. Die Zeigeeinrichtung kann z. B. eine Blende sein oder zumindest die Funktion einer Blende erfüllen. Z. B. kann die Zeigeeinrichtung zumindest axial fest, vorzugsweise auch drehfest mit dem Lagerelement verbunden sein. Im Grunde kann das Lagerelement die Zeigeeinrichtung bilden. Selbstverständlich ist es aber auch möglich, dass die Zeigeeinrichtung ein von dem Lagerelement separates Teil ist. Die Zeigeeinrichtung kann z. B. hülsenförmig sein.

In dieser Variante kann die Verschiebung des Lagerelements bewirken, dass im Bereich der Zeigeeinrichtung eine von der Dosisskala verschiedene Markierung erscheint, die an oder auf der Zeigeeinrichtung angeordnet oder gebildet ist. Beispielsweise kann die Zeigeeinrichtung innerhalb des Gehäuses angeordnet sein. Die Markierung der Zeigeeinrichtung kann in unbetätigtem Zustand der Antriebs- und Dosiervorrichtung von dem Gehäuse oder einem anderem Element verdeckt sein. Wird die Antriebs- und Dosiervorrichtung, insbesondere das Betätigungsglied, betätigt, so dass das Dosisanzeigeelement zusammen mit der Zeigeeinrichtung verschoben wird, kann die Markierung aus ihrer Abdeckung hervortreten, so dass die Markierung erblickbar oder ablesbar ist. Wird die Betätigung unterbrochen oder beendet, kann das Dosisanzeigeelement zusammen mit der Zeigeeinrichtung und dem Lagerelement in seine Ausgangsposition zurückverschoben werden, so dass die Markierung wieder unter der Abdeckung angeordnet ist.

Allgemein bevorzugt kann bei der Betätigung der Antriebs- und Dosiervorrichtung für eine Produktausschüttung eine Feder, insbesondere eine Kupplungs- oder Rückstellfeder gespannt werden. Mit anderen Worten ausgedrückt kann das Lagerelement bei der Betätigung gegen die Kraft einer insbesondere solchen Feder verschoben werden, insbesondere aus einer unbetätigten Position in eine betätigte Position. Die Feder kann z. B. eine Schrauben- oder Wendelfeder sein, die als Druckfeder wirkt. Diese Feder bewirkt ferner, dass beim Unterbrechen oder Beenden der Betätigung das Lagerelement in seine Ausgangsposition oder unbetätigte Position zurückgesetzt wird. Insbesondere wird das Lagerelement bei der Betätigung in distale Richtung verschoben. Mittels der Feder wird das Lagerelement in proximale Richtung zurück verschoben, wenn die Betätigung unterbrochen oder beendet wird.

Die Betätigung des Betätigungsglieds bewirkt insbesondere, dass das Lagerelement zusammen mit dem Dosisanzeigeelement relativ zu dem Gehäuse und entlang der Drehachse verschoben wird. Im weiteren Sinne kann die Betätigung des Betätigungsglieds bewirken, dass ein Vortriebsglied, dessen distales Ende vorgesehen ist, auf einen Kolben des an der Antriebs- und Dosiervorrichtung befestigten oder befestigbaren Produktbehälters zu wirken, in distale Richtung, insbesondere Ausschüttrichtung verschoben wird. Das Betätigungsglied kann z. B. an dem proximalen, d. h. hinteren Ende der Antriebs- und Dosiervorrichtung angeordnet sein oder das proximale Ende der Antriebs- und Dosiervorrichtung bilden. Alternativ kann das Betätigungselement seitlich am Gehäuse und/oder zwischen dem distalen Ende und dem proximalen Ende der Antriebs- und Dosiervorrichtung angeordnet sein. Allgemein kann das Betätigungsglied in der Art eines Betätigungsknopfs ausgebildet sein. Das Betätigungsglied wird bei der Betätigung vorzugsweise relativ zu dem Gehäuse oder dem Dosierglied verschoben. Insbesondere kann der Verwender der Vorrichtung das Betätigungsglied vorteilhaft z.B. mit dem Daumen seiner Hand, welche das Gehäuse der Antriebs- und Dosiervorrichtung umgreift, betätigen.

Das Betätigungsglied ist vorzugsweise mit dem Lagerelement so verbunden, dass es das Lagerelement bei Betätigung verschiebt, insbesondere über ein Kupplungsglied, das z. B. axialfest und drehbar mit dem Lagerelement verbunden sein kann.

In allgemein bevorzugten Ausführungen kann die Betätigung des Betätigungsglieds bewirken, dass das Dosisanzeigeelement relativ zu oder an dem Lagerelement oder dem Gehäuse gedreht, insbesondere geschraubt wird, insbesondere in eine Richtung, dass die sich bei der Drehbewegung an der Zeigeeinrichtung vorbei bewegenden Werte der Dosisskala zurückzählen. Vorzugsweise stehen der Drehwinkel des Dosisanzeigeelements und der Ausschütthub des Vortriebsglieds in einem proportionalen Zusammenhang, insbesondere zu jedem Zeitpunkt während der Dosisausschüttung. Hierdurch lässt sich eine Echtzeitanzeige verwirklichen, die bei der Dosisausschüttung zurückzählt, bis sie schließlich beim Wert 0 angelangt, wobei die Ausschüttung dieser Dosis dann beendet ist. Wird die Betätigung für die Ausschüttung während des Zurückdrehens des Dosisanzeigeelements unterbrochen, zeigt das Dosisanzeigeelement die noch für die Ausschüttung dieser Dosis erforderliche Restmenge an.

In einer Variante kann die Antriebs- und Dosiervorrichtung so ausgestaltet sein, dass die für das Zurückdrehen des Dosisanzeigeelements oder/und das Verschieben des Vortriebsglieds in distale Richtung benötigte Energie manuell, insbesondere durch eine auf das Betätigungsglied wirkende Kraft des Verwenders aufzubringen ist. Z. B. kann das Dosiseinstellglied, insbesondere der Dosierknopf aus dem proximalen Ende des Gehäuses für eine Dosiseinstellung herausgeschraubt werden, wobei er zur Dosisausschüttung unter Betätigung des Betätigungsglieds in das Gehäuse zurückgeschraubt wird.

In einer bevorzugten alternativen Variante kann die Antriebs- und Dosiervorrichtung so ausgestaltet sein, dass die für das Zurückdrehen des Dosisanzeigeelements oder/und das Verschieben des Vortriebsglieds in distale Richtung benötigte Energie automatisch, insbesondere durch eine in der Antriebs- und Dosiervorrichtung enthaltene Feder, insbesondere Ausschüttfeder, in der die benötigte Energie gespeichert ist oder werden kann, bereitgestellt wird. Z. B. kann die in der Ausschüttfeder gespeicherte Federenergie bei Betätigung des Betätigungsglieds an das Dosisanzeigeelement oder/und das Vortriebsglied abgegeben werden, so dass das Dosisanzeigeelement zurückgedreht und das Vortriebsglied in distale Richtung verschoben werden. Die Ausschüttfeder kann z. B. so mit dem Dosierglied gekoppelt sein, dass eine Drehung des Dosierglieds bei der Dosiseinstellung die Ausschüttfeder vorspannt. Die Feder kann dann die für die eingestellte Dosis erforderliche Energie speichern. Die Feder ist bei Auslieferung der Antriebs- und Dosiervorrichtung bereits mit so viel Energie vorgespannt sein, dass die Energie für mehrere Ausschüttungen der Produktdosis ausreicht, sprich für die Ausschüttung des gesamten aus dem Produktbehälter ausschüttbaren Produkts ausreicht. In dieser Alternative kann das Dosierglied bei der Dosiseinstellung von der Feder entkoppelt sein, d. h. nicht so mit der Ausschüttfeder gekoppelt sein, dass eine Drehung des Dosierglieds ein Spannen der Feder bewirkt. Hierdurch lässt sich das Dosierglied für den Verwender bei der Dosiseinstellung mit deutlich weniger Kraftaufwand drehen.

Das Dosierglied, insbesondere der Dosierknopf kann das Betätigungsglied, insbesondere den Betätigungsknopf umgeben oder aufnehmen. Somit können das Dosierglied und das Betätigungsglied das proximale Ende der Antriebs- und Dosiervorrichtung bilden. Das Betätigungsglied für die Betätigung ist relativ zu dem Dosierglied verschiebbar.

Die Antriebs- und Dosiervorrichtung weist ein Vortriebsglied auf, dessen distales Ende vorgesehen ist, auf einen Kolben, insbesondere mittelbar oder vorzugsweise unmittelbar zu wirken. Der Kolben kann Teil eines an der Antriebs- und Dosiervorrichtung befestigten oder befestigbaren Produktbehälters, wie z. B. einer Karpule sein. Das Vortriebsglied kann im weiteren Sinne als Kolbenstange bezeichnet werden, wobei das Vortriebsglied nicht notwendigerweise massiv sein muss, sondern auch hohl, wie z. B. hülsenförmig ausgestaltet sein kann. An dem distalen Ende des Vortriebsglieds kann optional ein z. B. drehbarer Flansch gebildet sein, der gegen den Kolben drückt. Allgemein ist es bevorzugt, dass das distale Ende des Vortriebsglieds gegen den Kolben drückt. Das Vortriebsglied ist vorzugsweise relativ zu dem Gehäuse entlang der Längsachse der Antriebs- und Dosiervorrichtung verschiebbar.

Die Antriebs- und Dosiervorrichtung weist ein Widerlager und vorzugsweise eine Führung auf, wobei das Vortriebsglied relativ zu dem Widerlager und vorzugsweise auch relativ zu der Führung in eine Richtung, insbesondere in distale Richtung oder in Ausschüttrichtung, bewegbar ist, um eine Ausschüttung der eingestellten Produktdosis zu bewirken. Vorzugsweise kann das Vortriebsglied mittels oder an der Führung, entlang der Längsachse der Antriebs- und Dosiervorrichtung gerade oder axial geführt sein. Das Vortriebsglied ist relativ zu dem Widerlager oder dem Gehäuse drehfest. - In einer alternativen Ausführungsform kann das Vortriebsglied relativ zu dem Widerlager oder dem Gehäuse kombiniert mit einer Längsbewegung drehbar, d. h. relativ zu dem Widerlager schraubbar sein, wenngleich weniger bevorzugt. Allgemein kann die Führung oder/und das Widerlager von dem Gehäuse, insbesondere einem hülsenförmigen Gehäuseteil oder einem z.B. hülsenförmigen, gehäusefesten Element gebildet werden.

Das Vortriebsglied und die insbesondere von dem Gehäuse gebildete Führung können zum Beispiel in einem unmittelbaren oder über ein Zwischenglied in einem mittelbaren Eingriff sein, der eine Drehung des Vortriebsglieds relativ zu dem Widerlager oder dem Gehäuse verhindert, aber eine Axialbewegung oder eine Schraubbewegung des Vortriebsglieds relativ zu dem Widerlager oder dem Gehäuse erlaubt. Die Führung kann z. B. eine Axialführung oder ein Gewinde mit einer nicht selbsthemmenden Gewindesteigung sein.

Die Führung oder der das Widerlager oder/und die Führung bildende Gehäuseabschnitt, insbesondere eine Innenhülse, kann vorzugsweise das Vortriebsglied umgeben, wie z. B. hülsenförmig umgeben und/oder gehäusefest sein oder von dem Gehäuse gebildet werden. Zwischen diesem hülsenförmigen Gehäuseteil und einem äußeren, vorzugsweise ebenfalls hülsenförmigen Gehäuseteil kann ein Ringspalt gebildet sein, der den Vorteil birgt, dass ein optional vorhandenes Dosisanzeigeelement, insbesondere Dosisanzeigetrommel, darin aufgenommen sein kann. Dies bewirkt, dass die Länge der Antriebs- und Dosiervorrichtung gering gehalten werden kann. Die Antriebs- und Dosiervorrichtung weist zwei zwischen dem Vortriebsglied und dem Widerlager wirkende Ausschüttfedern auf. Die erste Ausschüttfeder und die zweite Ausschüttfeder sind in Serie geschaltet. In Serie geschaltete Ausschüttfedern bedeutet, dass das distale Ende von einer aus erster und zweiter Ausschüttfeder gegen das proximale Ende der anderen aus erster und zweiter Ausschüttfeder drückt, und zwar mittelbar, über das Zwischenglied. Die erste Ausschüttfeder stützt sich an dem Widerlager und dem Zwischenglied und die zweite Ausschüttfeder an dem Zwischenglied und dem Vortriebsglied ab. Beispielsweise kann das distale Ende der ersten Ausschüttfeder distal des proximalen Endes der zweiten Ausschüttfeder angeordnet sein. Aufgrund des Zwischenglieds kann die Federkraft der ersten Feder von ihrem distalen Ende auf das proximale Ende der zweiten Ausschüttfeder übertragen werden. Insbesondere kann das Zwischenglied hülsenförmig sein und in einem Ringspalt zwischen erster und zweiter Ausschüttfeder angeordnet sein. In Serie geschaltete Ausschüttfedern erlauben, über einen verhältnismäßig langen Federweg eine verhältnismäßig gleich bleibende Federkraft. Die erste und zweite Ausschüttfeder sind jeweils eine Wendelfeder, die als Druckfeder wirkt. Die mindestens eine Ausschüttfeder, die eine erste und eine zweite wendelförmige Druckfeder umfasst, ist vorgespannt und wirkt so auf das Vortriebsglied, das sie versucht, das Vortriebsglied relativ zu dem Widerlager in distale Richtung, d. h. in Ausschüttrichtung zu verschieben. Die mindestens eine Ausschüttfeder ist im Auslieferungszustand der Antriebs- und Dosiervorrichtung insbesondere mit so viel Energie vorgespannt, dass sie die aus dem Produktbehälter maximal oder insgesamt ausschüttbare Produktmenge in mehreren Einzelausschüttungen d. h. insbesondere in mehreren Ausschüttungen einzelner Produktdosen ausschütten kann. Die Antriebs- und Dosiervorrichtung ist so ausgestaltet, dass nach jeder Einzelausschüttung oder Ausschüttung der Produktdosis, die nächste auszuschüttende Dosis neu eingestellt wird. Im Gegensatz zu Ausführungen, bei denen eine Ausschüttfeder bei jeder Dosiseinstellung neu vorgespannt wird, kann durch die mit der für die Ausschüttung der maximal aus dem Produktbehälter ausschüttbaren Produktmenge erforderlichen Energie vorgespannte Feder eine einfachere Dosiseinstellung erreicht werden, da das für die Dosiseinstellung relativ zu dem Gehäuse drehbare Dosierglied dann einfacher drehbar ist, weil die Feder bei der Dosiseinstellung nicht vorgespannt werden braucht. Dies erhöht den Anwendungskomfort für den Verwender der Vorrichtung.

Die Antriebs- und Dosiervorrichtung umfasst ferner ein Rotationsglied, dessen Drehung bewirkt, dass die Antriebsfeder Energie an das Vortriebsglied abgibt, wodurch das Vortriebsglied in distale Richtung bewegt wird. Das Rotationsglied übernimmt vorzugsweise die Funktion eines Steuerglieds, wobei die Drehung des Rotationsglieds um einen bestimmten Drehwinkel den Vorschub des Vortriebsglieds um einen bestimmten Ausschütthub bewirkt.

Durch selektive Freigabe oder Sperrung einer Drehung des Rotationsglieds relativ zu dem Gehäuse kann der Antriebsfeder erlaubt werden, dass sie das Vortriebsglied relativ zu dem Widerlager in distale Richtung bewegen kann oder nicht bewegen kann. Das Rotationsglied ist so mit dem Betätigungsglied gekoppelt dass es bei der Betätigung des Betätigungsglieds für eine Produktausschüttung für eine Drehung relativ zu dem Gehäuse freigegeben ist und bei Nichtbetätigung des Betätigungsglieds für eine Rotation relativ zu dem Gehäuse gesperrt ist. Insbesondere zwischen Betätigungsglied und Rotationsglied ist mindestens eine Kupplung angeordnet, welche die Freigabe und Sperrung der Drehung des Rotationsglieds relativ zu dem Gehäuse bewirkt.

Die mindestens eine Kupplung gibt durch Betätigen des Betätigungsglieds die Drehung des Rotationsglieds relativ zu dem Gehäuse frei und blockiert durch Loslassen des Betätigungsglieds die Drehung des Rotationsglieds relativ zu dem Gehäuse. Nach einem ersten Unteraspekt umfasst die mindestens eine Feder erfindungsgemäß eine erste wendelförmige Druckfeder und eine zweite wendelförmige Druckfeder, wobei sich die erste Druckfeder an dem Widerlager und einem Zwischenglied und die zweite Druckfeder an dem Zwischenglied und dem Vortriebsglied abstützen. Das Zwischenglied koppelt das Vortriebsglied drehfest mit dem Gehäuse oder dem Widerlager. Die erste Druckfeder stützt sich mit ihrem distalen Ende an dem Zwischenglied und ihrem proximalen Ende an dem Widerlager ab. Die zweite Druckfeder stützt sich mit ihrem distalen Ende an dem Vortriebsglied und mit ihrem proximalen Ende an dem Zwischenglied ab. Insbesondere sind die erste Druckfeder und die zweite Druckfeder in Serie geschaltet. Unter einer Druckfeder wird eine als Druckfeder wirkende wendelförmige Feder verstanden. Das Zwischenglied kann zum Beispiel ringförmig sein und das Vortriebsglied umgeben. Insbesondere kann das Zwischenglied in einem Ringspalt zwischen einer Innenhülse des Gehäuses, welche die Führung bildet, und dem Vortriebsglied angeordnet sein.

Insbesondere kann das Zwischenglied mit dem Gehäuse, insbesondere der Innenhülse und/oder der Führung in so einem Eingriff sein, dass das Zwischenglied relativ zu dem Gehäuse entlang der Längsachse verschiebbar und um die Längsachse verdrehfest ist. Das Zwischenglied kann mit dem Vortriebsglied in so einem Eingriff sein, dass das Zwischenglied relativ zu dem Vortriebsglied entlang der Längsachse verschiebbar und um die Längsachse verdrehfest ist. Hierdurch wird das Vortriebsglied über das Zwischenglied verdrehgesichert mit dem Gehäuse verbunden oder gekoppelt.

Das Zwischenglied befindet sich vorzugsweise in einer Schwimmstellung zwischen der ersten Feder und der zweiten Feder, d. h. dass die Position des Zwischenglieds so ist, dass die von der ersten Feder und von der zweiten Feder auf das Zwischenglied ausgeübten Kräfte sich aufgeben.

Beispielsweise kann die erste Druckfeder das Vortriebsglied umgeben und die zweite Druckfeder das Vortriebsglied umgeben. Das bedeutet, dass die erste Druckfeder und die zweite Druckfeder in dem Ringspalt zwischen der Innenhülse des Gehäuses und dem Vortriebsglied angeordnet sein können. Beispielsweise kann der Außendurchmesser der ersten Druckfeder größer sein als der Außendurchmesser der zweiten Druckfeder. Hierdurch kann mit der ersten Druckfeder hinsichtlich einer hohen Federkonstante und mit der zweiten Druckfeder hinsichtlich einer platzsparenden Bauweise optimiert werden.

Beispielsweise kann die erste Druckfeder das Vortriebsglied umgeben und das Vortriebsglied die zweite Druckfeder umgeben. Die erste Druckfeder kann in dem Ringspalt zwischen dem Gehäuse, insbesondere der Innenhülse, und dem Vortriebsglied angeordnet sein. Die zweite Druckfeder kann in dem Ringspalt zwischen Rotationsglied und Vortriebsglied angeordnet sein. Hierdurch lässt sich zumindest für den Bereich, in dem die zweite Druckfeder angeordnet ist, eine platzsparende Bauweise erreichen.

Das Zwischenglied kann sich durch einen länglichen und sich entlang der Längsachse erstreckenden Schlitz in der Wand des hülsenförmigen Vortriebsglieds erstrecken. Der Schlitz kann hierdurch gleichzeitig als Führung oder als Längsführung für das Zwischenglied an dem Vortriebsglied dienen. Die erste Feder kann sich an dem Abschnitt des Zwischenglieds abstützen, der sich außerhalb des Vortriebsglieds befindet, wobei sich die zweite Druckfeder an dem Abschnitt des Zwischenglieds abstützen kann, der sich innerhalb des Vortriebsglieds befindet. Das Zwischenglied ist bzw. wird während der Produktausschüttung in dem Schlitz verschiebbar bzw. verschoben.

Allgemein bevorzugt ist die zweite Druckfeder distal der ersten Druckfeder angeordnet. Insbesondere kann das proximale Ende der zweiten Druckfeder distal des distalen Endes der ersten Druckfeder angeordnet sein.

Die zweite Druckfeder kann vorzugsweise einen geringeren Außendurchmesser aufweisen als die Innenwand des Produktbehälters, an der der Kolben des Produktbehälters verschiebbar anliegt. Durch den geringen Außendurchmesser kann vorteilhaft erreicht werden, dass ein versehentlicher Kontakt der Druckfeder durch einen ausreichenden Abstand zur Innenwand vermieden wird.

Die zweite Druckfeder kann so angeordnet sein, dass sie sich in dem Produktbehälter befindet, wenn die Kolbenstange zur Ausschüttung des Produkts in dem Produktbehälter verschoben wird. Das Zwischenglied kann in Bezug auf den Produktbehälter hinsichtlich seiner Lage so abgestimmt sein, dass es entlang der Längsachse von einem proximalen Ende des Produktbehälters beabstandet ist oder das proximale Ende des Produktbehälters berührt, wenn die aus dem Produktbehälter maximal ausschüttbare Produktmenge ausgeschüttet ist. Wenn das Zwischenglied das proximale Ende des Produktbehälters während der Produktausschüttung nicht berührt, ist sichergestellt, dass beide Druckfedern zum Vortrieb des Kolbens beitragen. Wenn das Zwischenglied während der Produktausschüttung in einen Kontakt mit dem distalen Ende des Produktbehälters kommt, kann nur noch die zweite Druckfeder zur Ausschüttung beitragen, was in speziellen Anwendungen durchaus von Vorteil sein kann.

In einem zweiten Unteraspekt, der keinen Teil der Erfindung formt, kann das Vortriebsglied eine Schulter aufweisen, an der sich die mindestens eine Feder, insbesondere mit ihrem distalen Ende abstützt. Die mindestens eine Feder kann eine einzige Druckfeder, d. h. eine Wendelfeder, die als Druckfeder wirkt, sein. Ein sich distal der Schulter entlang der Längsachse erstreckende Abschnitt des Vortriebsglieds kann eine entlang der Längsachse gemessene Länge aufweisen, die größer ist als der zwischen dem proximalen Ende des Produktbehälters und dem Kolben bestehende Abstand, wenn der Kolben in der Position ist, in der die in dem Produktbehälter maximal ausschüttbare Produktmenge ausgeschüttet ist. In dieser Ausführungsform kann die Druckfeder nur in dem Bereich proximal der Schulter angeordnet sein, wobei in dem Bereich distal der Schulter keine weitere Feder angeordnet sein braucht. Hierdurch kann besonders vorteilhaft erreicht werden, dass der Bereich distal der Schulter platzsparend ausgelegt werden kann.

Insbesondere kann die Schulter in einem drehfesten und axial verschiebbaren Eingriff mit dem Gehäuse sein. Beispielsweise kann die Schulter eine Innenhülse des Vortriebsglieds und eine Außenhülse des Vortriebsglieds fest verbinden, wobei die mindestens eine Druckfeder in dem zwischen der Innenhülse und der Außenhülse gebildeten Ringspalt angeordnet sein kann oder ist. Beispielsweise kann die Außenhülse in einem drehfesten und axial verschiebbaren Eingriff mit dem Gehäuse, insbesondere der Innenhülse des Gehäuses oder der Führung sein. Hierdurch wird eine Drehung des Vortriebsglieds relativ zu dem Gehäuse verhindert, aber eine Verschiebung entlang der Längsachse erlaubt.

In einem dritten Unteraspekt, der keinen Teil der Erfindung formt, kann die mindestens eine Feder eine Torsionsfeder, insbesondere eine spiralförmig aus einem bandförmigen Material gewickelte Feder sein, die sich an dem Rotationsglied und dem Widerlager abstützt. Eine solche Feder kann als Spiral- oder Uhrenfeder bezeichnet werden. Die vorgespannte Torsionsfeder erzeugt ein Drehmoment zwischen Rotationsglied und dem Widerlager, wobei das Drehmoment versucht, dass Rotationsglied relativ zu dem Widerlager oder dem Gehäuse um die Längsachse zu verdrehen. Die Torsionsfeder und vorzugsweise auch das Widerlager für die Torsionsfeder, an dem sich die Torsionsfeder abstützt, kann distal eines Dosisanzeigeelements oder/und eines relativ zu dem Gehäuse entlang der Längsachse verschiebbare Lagerelements, welches mit einem Dosisanzeigeelement in einem Gewindeeingriff ist, angeordnet sein. Für die Ausgestaltung des Dosisanzeigeelements und des Lagerelements wird auf die übrige Beschreibung dieser Anmeldung verwiesen.

In dem dritten Unteraspekt kann das Rotationsglied drehfest und axial verschiebbar mit dem Vortriebsglied verbunden sein. Das Rotationsglied kann zum Beispiel hülsenförmig sein und das Vortriebsglied umgeben. Das Vortriebsglied und das Rotationsglied können so verbunden sein, dass das Rotationsglied in Bezug auf das Vortriebsglied drehfest und axial verschiebbar ist. Das Vortriebsglied kann dann zum Beispiel mit seinem Außengewinde in einem Gewindeeingriff mit dem Gehäuse oder einem gehäusefesten Element stehen. Bei Drehung des Rotationsglieds wird das Vortriebsglied an dem Gewinde des Gehäuses oder des gehäusefesten Elements entlang der Längsachse geschraubt, wodurch zum Bespiel die Produktausschüttung vorgenommen werden kann, d. h. der Kolben in dem Produktbehälter in distale Richtung verschoben werden kann.

Dadurch, dass das Rotationsglied das Vortriebsglieds umgibt, kann das Vortriebsglied zum Beispiel stangenförmig, insbesondere als Kolbenstange ausgeführt werden, wodurch es einen ausreichenden Abstand zur Innenwand des Produktbehälters einhält, wenn das Vortriebsglied in den Produktbehälter verschoben wird.

Allgemein bevorzugt kann der Drehwinkel des Rotationsglieds proportional zu dem Ausschütthub des Kolbens oder des Vortriebsglieds sein. Dies lässt sich durch die selektive Sperrung oder Freigabe des Rotationsglieds erreichen.

Vorteilhaft kann das Rotationsglied in einem Eingriff, insbesondere einem Gewindeeingriff mit dem Vortriebsglied sein. Durch die Gewindesteigung dieses Gewindeeingriffs wird erreicht, dass z. B. bei einer vollständigen Umdrehung des Rotationsglieds relativ zu dem Gehäuse das Vortriebsglied von der Ausschüttfeder um einen Hub verschiebbar ist, welcher der Gewindesteigung entspricht.

Z. B. können das Rotationsglied eine Gewindestange und das Vortriebsglied eine Gewindemutter aufweisen oder sein, wobei das Gewinde der Gewindemutter in das Gewinde der Gewindestange eingreift.

In einem alternativen Beispiel können das Rotationsglied eine Gewindemutter und das Vortriebsglied eine Gewindestange aufweisen oder sein, wobei das Gewinde der Gewindemutter in das Gewinde der Gewindestange eingreift.

Vorzugsweise ist das Rotationsglied axial fest in Bezug auf das Gehäuse oder kann sich zumindest in eine, vorzugsweise in distaler Richtung axialfest an dem Gehäuse oder einem gehäusefesten Element, wie z. B. dem Widerlager abstützen.

Es ist vorteilhaft, dass das Rotationsglied während des Einstellens einer Dosis, d. h. im unbetätigten Zustand insbesondere mittels der mindestens einen Kupplung, insbesondere einer ersten Kupplung drehfest mit dem Gehäuse verbunden ist und während der Betätigung der Vorrichtung zum Ausschütten der Produktdosis relativ zu dem Gehäuse gedreht wird oder drehbar ist. Das Dosisanzeigeelement, insbesondere die Dosisanzeigetrommel kann optional in einem Gewindeeingriff mit dem Gehäuse oder einem gehäusefest angeordneten Element stehen.

Das Dosisanzeigeelement, insbesondere die Dosisanzeigetrommel kann während des Einstellens einer Dosis, d. h. im unbetätigten Zustand der Antriebs- und Dosiervorrichtung oder des Betätigungsglieds relativ zu dem Rotationsglied drehbar sein. Vorzugsweise ist das Dosisanzeigeelement während der Betätigung der Vorrichtung zum Ausschütten der Produktdosis relativ zu dem Rotationsglied drehfest und z.B. axial bewegbar, oder drehfest mit dem Rotationsglied verbunden, insbesondere mit der mindestens einen Kupplung, insbesondere einer zweiten Kupplung.

Vorteilhaft wird bewirkt, dass bei der Dosisausschüttung, d. h. bei der Betätigung des Betätigungsglieds, die Ausschüttfeder das Dosisanzeigeelement in seine Nulldosisposition zurückschraubt, insbesondere über das Rotationsglied und vorzugsweise über ein Kupplungsglied, welches vorzugsweise drehfest aber axial verschiebbar in Bezug auf das Dosisanzeigeelement angeordnet ist. Insbesondere können das Kupplungsglied und das Dosisanzeigeelement in einem drehfesten Eingriff sein, der eine Axialbewegung zwischen Dosisanzeigeelement und Kupplungsglied zulässt. Dieser Eingriff kann z. B. mittels einer Längsführung bewirkt werden. Bevorzugt ist das Kupplungsglied axial fest aber drehbar mit dem Lagerelement verbunden.

Die erste Kupplung kann kinematisch zwischen dem Gehäuse, insbesondere dem Lagerelement, und dem Rotationsglied angeordnet sein. Die erste Kupplung ist während der Einstellung der Produktdosis oder bei unbetätigtem Betätigungsglied eingekuppelt, wodurch das Rotationsglied relativ zu dem Gehäuse verdrehfest ist. Während der Produktausschüttung oder bei betätigtem Betätigungsglied ist die erste Kupplung ausgekuppelt, wodurch das Rotationsglied relativ zu dem Gehäuse verdrehbar ist.

Die erste Kupplung kann eine erste Kupplungsstruktur, die verdrehfest mit dem Gehäuse verbunden ist, insbesondere von dem Gehäuse oder dem Lagerelement gebildet wird, und eine zweite Kupplungsstruktur, die verdrehfest mit dem Rotationsglied verbunden ist, insbesondere von dem Rotationsglied gebildet wird, aufweisen. Die erste Kupplungsstruktur kann eine Verzahnung sein oder aufweisen, wobei die zweite Kupplungsstruktur eine Verzahnung sein oder aufweisen kann. Die Verzahnungen der ersten Kupplungsstruktur und der zweiten Kupplungsstruktur können bei eingekuppelter erster Kupplung formschlüssig ineinandergreifen. Die erste Kupplungsstruktur kann eine Außenverzahnung sein, wobei die zweite Kupplungsstruktur eine Innenverzahnung sein kann. Alternativ kann die erste Kupplungsstruktur eine Innenverzahnung sein, wobei die zweite Kupplungsstruktur eine Außenverzahnung sein kann.

Die zweite Kupplung kann zwischen einem hülsenförmigen Kupplungsglied, welches zum Beispiel drehbar und axialfest mit dem Lagerelement verbunden, insbesondere in einem Eingriff sein kann, und dem Rotationsglied angeordnet sein. Die zweite Kupplung ist während der Einstellung der Produktdosis oder bei unbetätigtem Betätigungsglied ausgekuppelt, wodurch dem Kupplungsglied erlaubt wird, relativ zu dem Rotationsglied verdreht zu werden. Während der Produktausschüttung oder bei betätigtem Betätigungsglied ist die zweite Kupplung eingekuppelt. Hierdurch wird insbesondere erreicht, dass das Rotationsglied und das Kupplungsglied verdrehfest verbunden sind. Das Kupplungsglied kann somit von dem Rotationsglied mitgedreht werden. Die zweite Kupplung kann eine dritte Kupplungsstruktur, welche verdrehgesichert mit dem Kupplungsglied verbunden ist, insbesondere von dem Kupplungsglied gebildet wird, und eine vierte Kupplungsstruktur, welche verdrehfest mit dem Rotationsglied verbunden ist, insbesondere von dem Rotationsglied gebildet wird, umfassen. Die dritte Kupplungsstruktur kann eine Verzahnung sein oder aufweisen, wobei die vierte Kupplungsstruktur eine Verzahnung sein oder aufweisen kann. Die Verzahnungen der dritten und vierten Kupplungsstruktur können formschlüssig ineinandergreifen, wenn die zweite Kupplung eingekuppelt ist. Zum Beispiel kann die dritte Kupplungsstruktur eine Innenverzahnung sein, wobei die vierte Kupplungsstruktur eine Außenverzahnung sein kann. Alternativ kann die dritte Kupplungsstruktur eine Außenverzahnung sein, wobei die vierte Kupplungsstruktur eine Innenverzahnung sein kann.

Grundsätzlich können die Verzahnungen für die zweite Kupplungsstruktur und die vierte Kupplungsstruktur voneinander separate Verzahnungen sein. Vorzugsweise können die zweite Kupplungsstruktur und die vierte Kupplungsstruktur von einer gemeinsamen Verzahnung gebildet werden.

Insbesondere können die erste und die zweite Kupplung so aufeinander abgestimmt sein, dass das Betätigungsglied zwischen der unbetätigten Position und der betätigten Position eine, zum Beispiel erste, Zwischenposition einnehmen kann, in welcher die erste Kupplung eingekuppelt ist und die zweite Kupplung eingekuppelt ist. Dies bewirkt vorteilhaft, dass die Drehung des Kupplungsglieds durch die Antriebsfeder erst dann freigegeben wird, wenn sichergestellt ist, dass das Rotationsglied drehfest mit dem Kupplungsglied gekoppelt ist. Hierdurch wird vorteilhaft eine Fehlfunktion der Vorrichtung vermieden, die auftreten könnte, wenn die zweite Kupplung noch nicht eingekuppelt ist und die erste Kupplung bereits ausgekuppelt ist.

Die Antriebs- und Dosiervorrichtung weist vorzugsweise eine dritte Kupplung auf, welche zwischen dem Dosiseinstellglied und dem Kupplungsglied angeordnet ist. Die dritte Kupplung ist während der Einstellung der Produktdosis oder bei unbetätigtem Betätigungsglied eingekuppelt, wodurch das Kupplungsglied und das Dosiseinstellglied in Bezug zueinander verdrehgesichert sind. Während der Produktausschüttung oder bei betätigtem Betätigungsglied ist die dritte Kupplung ausgekuppelt, wodurch das Kupplungsglied mittels der vorgespannten Antriebsfeder relativ zu dem Dosiseinstellglied drehbar ist.

Die dritte Kupplung kann eine fünfte Kupplungsstruktur, die verdrehfest mit dem Dosiseinstellglied verbunden ist, insbesondere von dem Dosiseinstellglied gebildet wird, und eine sechste Kupplungsstruktur, die verdrehfest mit dem Kupplungsglied verbunden ist, insbesondere von dem Kupplungsglied gebildet wird, aufweisen. Die fünfte Kupplungsstruktur kann ein Verzahnung sein oder aufweisen, wobei die sechste Kupplungsstruktur eine Verzahnung sein oder aufweisen kann. Die Verzahnungen der fünften Kupplungsstruktur und der sechsten Kupplungsstruktur können formschlüssig ineinandergreifen, wenn die dritte Kupplung eingekuppelt ist. Die fünfte Kupplungsstruktur kann eine Innenverzahnung sein, wobei die sechste Kupplungsstruktur eine Außenverzahnung sein kann. Alternativ kann die fünfte Kupplungsstruktur eine Außenverzahnung sein, wobei die sechste Kupplungsstruktur eine Innenverzahnung sein kann.

Insbesondere können die erste und die dritte Kupplung so aufeinander abgestimmt sein, dass das Betätigungsglied zwischen der unbetätigten Position und der betätigten Position eine, zum Beispiel zweite, Zwischenposition einnehmen kann, in welche die erste Kupplung eingekuppelt und die dritte Kupplung eingekuppelt sind.

Vorzugsweise kann die Antriebs- und Dosiervorrichtung eine vierte Kupplung aufweisen, welche zwischen, insbesondere kinematisch zwischen dem Dosiseinstellglied und dem Gehäuse angeordnet ist. Die vierte Kupplung ist während der Einstellung der Produktdosis oder bei unbetätigtem Betätigungsglied ausgekuppelt, wodurch das Dosiseinstellglied relativ zu dem Gehäuse verdrehbar ist. Während der Produktausschüttung oder bei betätigtem Betätigungsglied ist die vierte Kupplung eingekuppelt, wodurch das Dosiseinstellglied relativ zu dem Gehäuse verdrehfest ist. Allgemein ist bevorzugt, dass das Dosiseinstellglied während der Produktausschüttung relativ zu dem Gehäuse verdrehfest ist.

Die dritte Kupplung kann eine siebte Kupplungsstruktur, die verdrehfest mit dem Gehäuse verbunden ist, insbesondere von dem Gehäuse gebildet wird und eine achte Kupplungsstruktur, die verdrehfest mit dem Dosiseinstellglied verbunden ist, insbesondere von dem Dosiseinstellglied oder dem Betätigungsglied, welches zum Beispiel permanent drehfest mit dem Dosiseinstellglied verbunden sein kann, gebildet wird, aufweisen. Die siebte Kupplungsstruktur kann eine Verzahnung sein oder aufweisen, wobei die achte Kupplungsstruktur eine Verzahnung sein oder aufweisen kann. Die Verzahnungen der siebten Kupplungsstruktur und der achten Kupplungsstruktur können bei eingekuppelter dritter Kupplung formschlüssig ineinandergreifen. Die siebte Kupplungsstruktur kann eine Außenverzahnung sein, wobei die achte Kupplungsstruktur eine Innenverzahnung sein kann. Alternativ kann die siebte Kupplungsstruktur eine Innenverzahnung sein, wobei die achte Kupplungsstruktur eine Außenverzahnung sein kann.

In allgemein bevorzugten Ausführungen kann das Dosisanzeigeelement einen Anschlag, wie z. B. einen Nulldosisanschlag aufweisen, der von einem Gegenanschlag, insbesondere einem Nulldosisgegenanschlag wegbewegt wird, wenn eine Dosiserhöhung vorgenommen wird, und der zu dem Gegenanschlag hinbewegt wird, wenn eine Dosisverringerung vorgenommen wird, oder wenn die Vorrichtung für die Ausschüttung der eingestellten Produktdosis betätigt wird.

Insbesondere kann das Dosisanzeigeelement während des Einstellens der Produktdosis, d. h. bei Dosiserhöhung und Dosisverringerung, von dem Rotationsglied zumindest rotatorisch entkoppelt sein und bei der Betätigung der Vorrichtung zum Ausschütten der Produktdosis so mit dem Rotationsglied gekoppelt sein, dass eine Drehung des Rotationsglieds bewirkt, dass das Dosisanzeigeelement zu dem Gegenanschlag, d. h. der Nulldosisanschlag zu dem Nulldosisgegenanschlag hin bewegt wird. Sind der Nulldosisanschlag und der Nulldosisgegenanschlag in einem Anschlag oder einem Kontakt, wird hierdurch und insbesondere über die zweite Kupplung, eine Drehung des Rotationsglieds und somit ein weiterer Vorschub des Vortriebsglieds relativ zu dem Gehäuse verhindert.

In vorteilhaften Weiterbildungen kann die Antriebs- und Dosiervorrichtung einen Mechanismus zum Verhindern des Einstellens einer Dosis, welche die Menge eines Medikaments in dem Produktbehälter übersteigt, aufweisen. Insbesondere kann dieser Mechanismus die Drehung des Dosierglieds in eine Richtung, welche eine Dosiserhöhung bewirken würde blockieren, insbesondere auch wenn der Maximaldosisanschlag des Dosisanzeigeelements und der Maximaldosisgegenanschlag noch nicht in einem Eingriff sind oder wenn in der Zeigeeinrichtung eine Dosis angezeigt wird, die kleiner ist als die maximal einstellbare Produktdosis. Der Mechanismus verhindert somit, dass eine Dosis eingestellt werden kann, welche die Restmenge des in dem Produktbehälters enthaltenen Produkts übersteigt, wodurch die Gefahr einer Fehlanwendung der Antriebs- und Dosiervorrichtung verringert wird. Der Mechanismus kann z. B. einen Begrenzer aufweisen, der zwischen zwei Teilen angebracht ist, von denen sich eines relativ zu dem anderen während der Dosiseinstellung dreht und bei der Betätigung, d. h. der Dosisausschüttung nicht dreht. Z. B. kann der Begrenzer zwischen dem Dosiseinstellglied, das insbesondere als Dosiseinstellknopf oder Dosiseinstellhülse ausgestaltet sein kann, und dem Gehäuse oder einem gehäusefesten Element angeordnet sein. Der Begrenzer, das Dosiseinstellglied und das Gehäuse können so miteinander gekoppelt sein, dass eine relative Drehung, insbesondere während der Dosiseinstellung, zwischen dem Dosiseinstellglied und dem Gehäuse bewirkt, dass sich der Begrenzer zu einer Stoppposition hinbewegt, in welcher der Begrenzer das Einstellen einer Dosis verhindert, die die Menge eines Produkts in dem Produktbehälter übersteigt. Beispiele für entsprechend geeignete Begrenzer werden in der WO 2010/149209 oder in der WO 01/19434 A1, insbesondere in deren Figur 3 offenbart. Beispielsweise kann der Begrenzer ein Innengewinde aufweisen, welches in dem Eingriff mit einem Außengewinde des Gehäuses ist. Insbesondere kann der Begrenzer an seiner Außenseite eine Längsführung aufweisen, mit der er in einem Eingriff mit dem Dosiseinstellglied ist, so dass das Dosiseinstellglied relativ zu dem Begrenzer drehfest ist. Alternativ kann das Gehäuse die Längsführung für den Begrenzer aufweisen, so dass der Begrenzer relativ zu dem Gehäuse drehfest ist und kann der Begrenzer ein Gewinde, insbesondere Außengewinde aufweisen, welches in einen Gewinde, insbesondere Innengewinde des Dosiseinstellglieds eingreift.

Die Stoppposition wird durch einen Anschlag für den Begrenzer definiert, wobei der Anschlag von dem Gehäuse oder dem Dosiseinstellglied oder einem zumindest axial oder in Umfangsrichtung gehäusefesten Mittel gebildet werden kann. Sind der Begrenzer und der Anschlag in einem Kontakt, ist eine Drehung des Dosiseinstellglieds in eine Drehrichtung, welche eine Erhöhung der Dosis bewirken würde, nicht mehr möglich oder blockiert.

In allgemein bevorzugten Weiterbildungen kann die Antriebs- und Dosiervorrichtung mindestens einen Signalerzeugungsmechanismus aufweisen, der angepasst ist, während der Dosiseinstellung oder/und der Produktausschüttung ein akustisches und/oder taktiles Signal, insbesondere mechanisch, zu erzeugen. Ein solches Signal kann insbesondere als Klicksignal wahrgenommen werden. Zum Beispiel kann ein (erster) Signalerzeugungsmechanismus vorgesehen sein, der das Signal während der Dosiseinstellung erzeugt und optional als Dosierklickeinrichtung bezeichnet werden kann. Ferner kann ein weiterer (zweiter) Signalerzeugungsmechanismus vorgesehen sein, der das Signal während der Produktausschüttung erzeugt und optional als Ausschüttklickeinrichtung bezeichnet werden kann. Alternativ kann ein (gemeinsamer) Signalerzeugungsmechanismus vorgesehen sein, der das Signal während der Dosiseinstellung und während der Produktausschüttung erzeugt.

Der Signalerzeugungsmechanismus kann allgemein zwischen zwei Teilen angeordnet sein, die sich bei der Dosiseinstellung oder/und der Produktausschüttung relativ zueinander bewegen, insbesondere drehen. Eines der Teile kann ein z.B. federnd angeordnetes Rastglied aufweisen, das in eine z. B. über den Umfang angeordnete Verzahnung des anderen der zwei Teile eingreift. Wird ein Teil relativ zu dem anderen verdreht, kann das Rastglied über die Verzahnung gleiten und dabei das Signal erzeugen. Die Verzahnung kann von einem Innenumfang oder einem Außenumfang oder einer Stirnfläche des Teils gebildet sein.

Der Signalerzeugungsmechanismus für die Dosiseinstellung kann insbesondere zwischen Kupplungsglied und Lagerelement oder Rotationsglied gebildet sein. Vorzugsweise dreht sich das Kupplungsglied während der Dosiseinstellung relativ zu dem Lagerelement bzw. dem Rotationsglied, wodurch ein Signalerzeugungsmechanismus gebildet wird, der das Signal während der Dosiseinstellung erzeugt. Das Kupplungsglied kann das Rastglied und das Rotationsglied oder das Lagerelement kann die Verzahnung, in welche das Rastglied eingreift, bilden. Die Verzahnung kann z. B. die Verzahnung sein, welche die zweite und/oder vierte Kupplungsstruktur bildet.

Der Signalerzeugungsmechanismus für die Produktausschüttung kann insbesondere zwischen Kupplungsglied und Betätigungsglied oder Dosierglied gebildet sein. Zum Beispiel kann das Betätigungsglied die Verzahnung, insbesondere Innenverzahnung, und das Kupplungsglied oder ein verdrehfest mit dem Kupplungsglied verbundener Klicker das Rastglied bilden. Vorzugsweise dreht sich das Kupplungsglied, während, insbesondere nur während der Produktausschüttung relativ zu dem Betätigungsglied oder dem Dosisglied, wodurch ein Signalerzeugungsmechanismus gebildet wird, der das Signal während der Produktausschüttung erzeugt.

Die Erfindung wurde anhand mehrerer vorteilhafter Ausführungsformen beschrieben. Im Folgenden werden vorteilhafte Ausführungsformen anhand von Figuren beschrieben. Die dabei offenbarten Merkmale bilden den Gegenstand der Ansprüche, insbesondere der unabhängigen Ansprüche, insbesondere einzeln und in jeglicher Kombination mit den Ansprüchen oder/und der vorhergehenden Beschreibung vorteilhaft weiter. Es zeigen:
- Figur 1: verschiedene Darstellungen einer ersten Ausführungsform einer erfindungsgemäßen Antriebs- und Dosiervorrichtung,
- Figur 2: verschiedene Darstellungen einer zweiten Ausführungsform einer erfindungsgemäßen Antriebs- und Dosiervorrichtung,
- Figur 3: verschiedene Darstellungen einer dritten Ausführungsform einer nicht erfindungsgemäßen Antriebs- und Dosiervorrichtung und
- Figur 4: verschiedene Darstellungen einer vierten Ausführungsform einer nicht erfindungsgemäßen Antriebs- und Dosiervorrichtung.

Die Antriebs- und Dosiervorrichtung umfasst gemäß der ersten bis vierten Ausführungsform, ein hülsenförmiges Gehäuse 4, welches eine Außenhülse 4b aufweist, welche vom Verwender mit einer Hand umgreifbar ist. Das Gehäuse 4 umfasst ferner eine Innenhülse 4a, die ein Widerlager 4i bildet und konzentrisch zu der Außenhülse 4b angeordnet ist. Innenhülse 4a und Außenhülse 4b sind über einen Ringsteg miteinander verbunden. Zwischen der Außenhülse 4b und der Innenhülse 4a ist ein Ringspalt gebildet, in dem ein Dosisanzeigeelement 10, das insbesondere als Dosisanzeigetrommel, d. h. hülsenförmig ausgestaltet ist, ein Lagerelement 9 und ein Kupplungsglied 2, das hülsenförmig ist und insbesondere auch als Anzeigekupplung bezeichnet werden kann, angeordnet sind.

An dem distalen Ende des Gehäuses 4 ist eine hülsenförmige Produktbehälteraufnahme 5 aus einem vorzugsweise transparenten Material angeordnet, in der ein Produktbehälter 14 in der Gestalt einer Karpule aufgenommen ist. Der Produktbehälter 14 ist mittels der Produktbehälteraufnahme 5 mit dem Gehäuse 4 unlösbar verbunden, so dass die Antriebs- und Dosiervorrichtung insbesondere zusammen mit der Produktbehälteraufnahme 5 und dem Produktbehälter 14 eine Einweg-Injektionsvorrichtung bildet, die nach vollständiger Entleerung des Produktbehälters 14 als Ganzes entsorgt wird. Der Produktbehälter 14 weist an seinem distalen Ende ein Septum 14b auf, welches von einer durch am distalen Ende des Produktbehälters 14 bzw. der Produktbehälteraufnahme 5 anbringbaren Nadel durchstechbar ist. In dem Produktbehälter 14 ist ein Kolben 14a aufgenommen, wobei das auszuschüttende Produkt zwischen dem Septum 14b und dem Kolben 14a angeordnet ist. Eine Verschiebung des Kolbens 14a in Richtung Septum oder in distale Richtung, mithin Ausschüttrichtung, bewirkt eine Ausschüttung des in dem Produktbehälter 14 enthaltenen Produkts. Es kann eine Schutzkappe (nicht gezeigt) vorgesehen sein, welche über der Produktbehälteraufnahme 5 anbringbar ist und vor dem Injizieren einer Dosis abgenommen wird.

Die Antriebs- und Dosiervorrichtung weist ein Vortriebsglied 8 und ein Rotationsglied 1 auf. Das Vortriebsglied 8 ist so angeordnet, dass ein distales Ende 8g auf den Kolben 14a wirken, insbesondere gegen den Kolben 14a drücken kann. Das Rotationsglied 1 ist relativ zu dem Gehäuse 4 axialfest und um die Längsachse L drehbar angeordnet. Das Rotationsglied 1 und das Vortriebsglied 8 sind so miteinander gekoppelt bzw. greifen so ineinander, dass eine Drehung des Rotationsglieds 1 relativ zu dem Gehäuse 4 und/oder zu dem Vortriebsglied 8 eine Bewegung des Vortriebsglieds 8 entlang der Längsachse L in distale Richtung zur Verschiebung des Kolbens 14a bewirkt.

Das Rotationsglied 1 weist an seinem proximalen Ende einen vergrößerten Durchmesser, insbesondere in der Gestalt eines verbreiterten Kopfs auf. An dem Kopf sind parallel zur Längsachse L eine Außenverzahnung bildende Zähne angeordnet, die als zweite und/oder vierte Kupplungsstruktur 1b dienen, wie weiter unten beschrieben wird. An dem Kopf ist eine ringförmige, vom Durchmesser her gegenüber dem Kopf verkleinerte Reibfläche angeordnet, die in einem Kontakt mit einem nach innen ragenden, zum Beispiel ein Widerlager 4i bildenden Ringsteg des Gehäuses 4 ist. Durch den verringerten Durchmesser der ringförmigen Reibfläche wird der Angriffspunkt der resultierenden Kraft näher zur Längsachse L hin verschoben, wodurch das Reibmoment zwischen Rotationsglied 1 und Gehäuse 4 herabgesetzt wird.

Die Antriebs- und Dosiervorrichtung weist mindestens eine Ausschüttfeder 11; 11a, 11b auf. Die mindestens eine Ausschüttfeder 11; 11a, 11b ist bei der Auslieferung, d. h. im Auslieferungszustand der Antriebs- und Dosiervorrichtung so stark vorgespannt, dass die in ihr gespeicherte Energie ausreicht, das in dem Produktbehälter 14 enthaltene Produkt im Wesentlichen vollständig auszuschütten, insbesondere mit mehreren Einzelausschüttungen, zwischen den jeweils eine neue Dosiseinstellung vorgenommen wird. Der Vorteil der mindestens einen vorgespannten Feder 11; 11a, 11b ist, dass die mindestens eine Feder 11; 11a, 11b zum Beispiel nicht während der Dosiseinstellung gespannt werden muss, wodurch für den Verwender der Vorrichtung eine kraftsparende, d. h. einfachere Dosiseinstellung vornehmbar ist.

Die mindestens eine Ausschüttfeder 11; 11a, 11b ist als Schrauben- oder Wendelfeder gebildet, die als Druckfeder wirkt und danach strebt, das Widerlager 4i und das Vortriebsglied 8 auseinander zu drücken, d. h. das Vortriebsglied 8 in die distale Richtung relativ zu dem Gehäuse 4 zu verschieben.

Das Gehäuse 4, insbesondere die Innenhülse 4a und ein hülsenförmiges Vortriebsglied 8, welches auch als Stößel bezeichnet werden kann, sind relativ zueinander drehfest und axial verschiebbar gekoppelt. Das Vortriebsglied 8 ist relativ zu dem Gehäuse 4 drehfest und axial entlang der Längsachse L verschiebbar.

Das Rotationsglied 1 ist als Gewindestange ausgebildet, die ein Außengewinde 1a bildet und mit einem Innengewinde des hülsenförmigen Vortriebsglieds 8 in einem Gewindeeingriff ist. Die Steigung der Gewinde des Gewindeeingriffs zwischen dem Vortriebsglied 8 und dem Rotationsglied 1 ist so groß, dass keine Selbsthemmung des Gewindeeingriffs auftritt, d. h., dass das Rotationsglied 1 aufgrund der entlang der Längsachse L wirkenden Axialkraft der Ausschüttfeder 11 relativ zu dem Vortriebsglied 8 um die Längsachse L drehbar oder rotierbar ist.

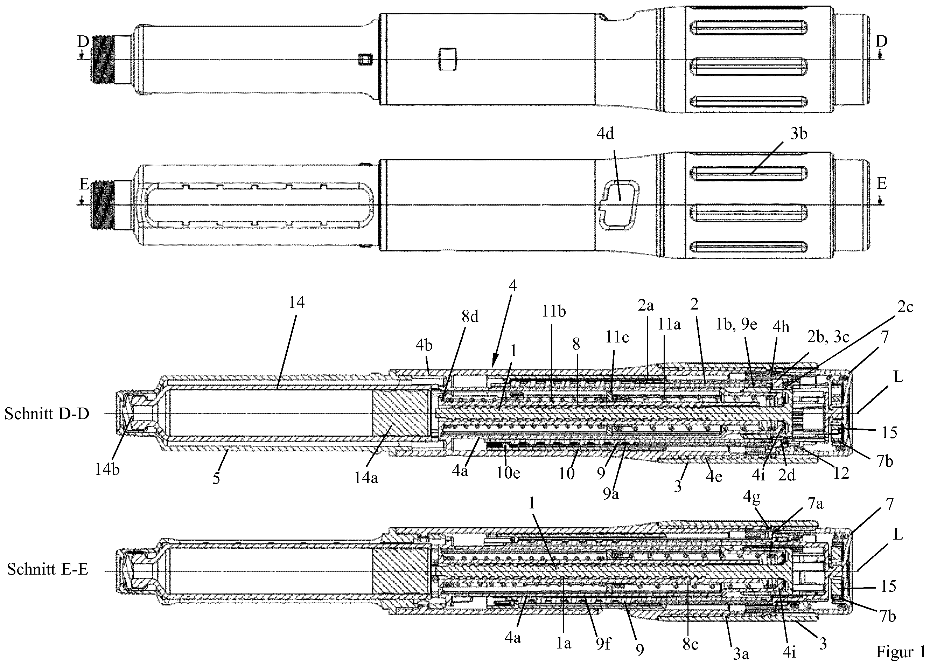

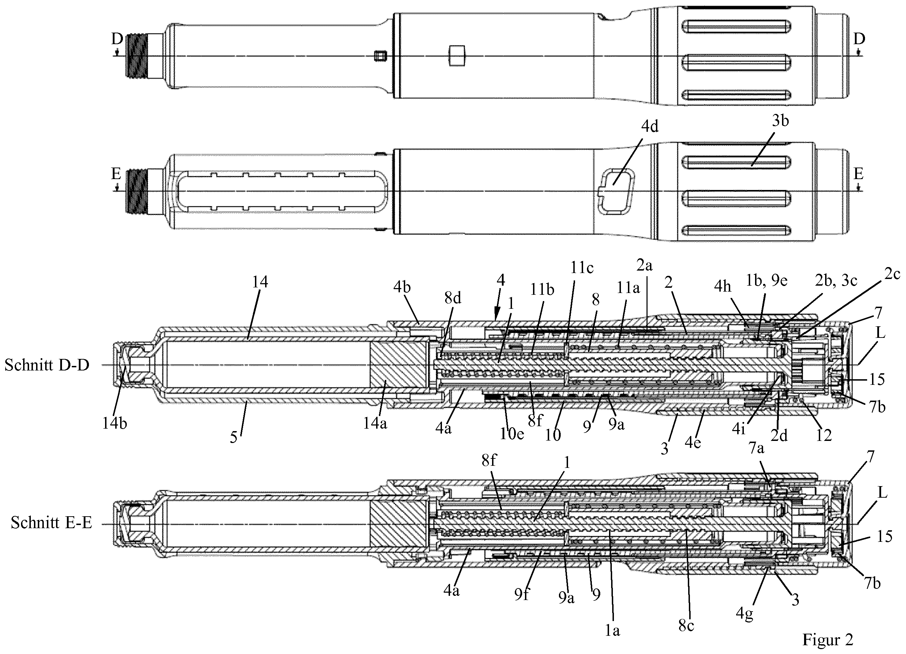

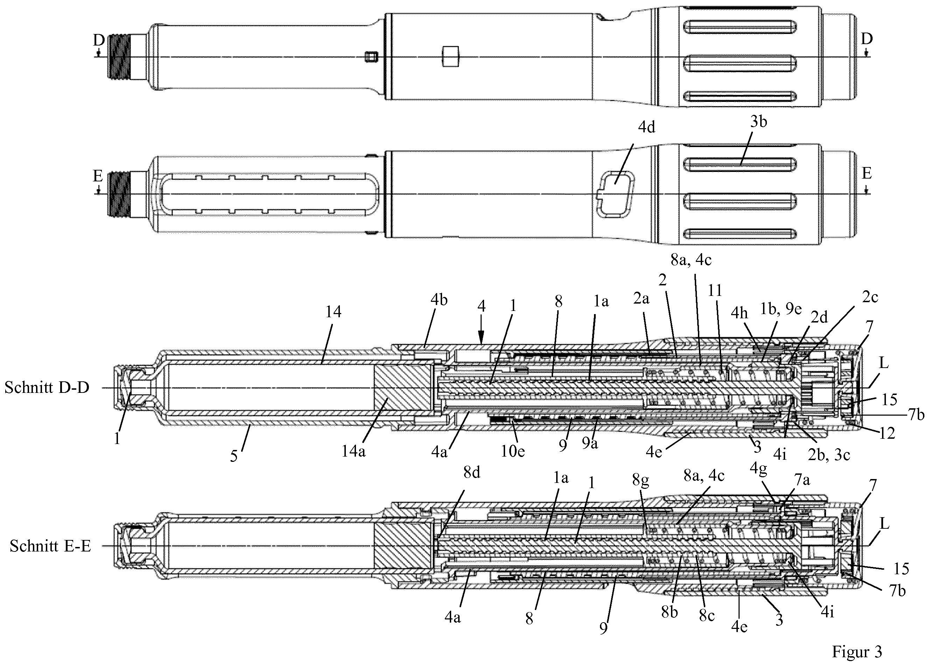

Das Gehäuse 4, insbesondere das proximale Ende der Innenhülse 4a bildet das Widerlager 4i für die mindestens eine Ausschüttfeder 11; 11a, 11b, die sich an dem Widerlager 4i und dem Bereich des distalen Endes des Vortriebsglieds 8 abstützt. Die mindestens eine Feder 11; 11a, 11b ist in der dritten Ausführungsform (Figur 3) eine einzige Feder und in der ersten und zweiten Ausführungsform (Figuren 1 und 2) eine erste Feder 11a und eine zweite Feder 11b. Die erste Feder 11a und die zweite Feder 11b sind in Serie geschaltet.

In der dritten Ausführungsform, die keinen Teil der Erfindung formt, stützt sich die Feder 11 mit ihrem distalen Ende an einem Ringsteg, insbesondere die Schulter 8g, des Vortriebsglieds 8 ab, der eine Außenhülse und eine Innenhülse 8b des Vortriebsglieds 8 fest verbindet. Die Feder 11 stützt sich mit ihrem proximalen Ende an dem von Gehäuse 4 gebildeten und nach innen ragenden Ringsteg ab, welcher das Widerlager 4i bildet. Die Feder 11 ist in dem Ringspalt zwischen der Außenhülse und der Innenhülse 8b des Vortriebsglieds 8 zumindest teilweise angeordnet. Die Innenhülse 8b des Vortriebsglieds 8 weist das Innengewinde 8c für den Gewindeeingriff mit dem Rotationsglied 1 auf. Das Vortriebsglied 8, insbesondere dessen Außenhülse und das Gehäuse 4, insbesondere dessen Innenhülse 4a greifen so ineinander, dass das Vortriebsglied 8 relativ zu dem Gehäuse 4 um die Längsachse L verdrehfest ist und entlang der Längsachse L verschiebbar ist. Zwischen der Innenhülse 4a und dem Vortriebsglied 8 bzw. deren Außenhülse ist eine Führung mittels mindestens einer Längsrippe 8a und mindestens einer Längsführung 4c gebildet, welche eine Drehung des Vortriebsglieds 8 relativ zu dem Gehäuse 4 verhindert und eine Axialbewegung des Vortriebsglieds 8 relativ zu dem Gehäuse 4 erlaubt. Die Längsrippe 8a wird vorzugsweise von der Außenhülse des Vortriebsglieds 8 gebildet. Das Vortriebsglied 8 weist die Innenhülse 8b auf, welche in diesem Beispiel an ihrem proximalen Ende das Innengewinde 8a aufweist, welches in das Außengewinde 1a des als Gewindestange ausgestalteten Rotationsglieds 1 eingreift.

Der Ringsteg, insbesondere die Schulter 8g, des Vortriebsglieds 8 ist bezogen auf die entlang der Längsachse L gemessene Gesamtlänge des Vortriebsglieds 8 in der proximalen Hälfte, insbesondere in dem proximalen Drittel des Vortriebsglieds 8 angeordnet. Der entlang der Längsachse L gemessene Abstand zwischen dem distalen Ende des Vortriebsglieds 8 und dem Ringsteg des Vortriebsglieds 8 ist vorzugsweise größer als der entlang der Längsachse L gemessene Abstand zwischen dem Kolben 14a und dem proximalen Ende des Produktbehälters 14, wenn der Kolben 14a in der Position ist, in der die aus dem Produktbehälter maximal ausschüttbare Produktmenge ausgeschüttet ist, d. h. bei im Wesentlichen vollständig entleerten Produktbehälter. Hierdurch wird vorteilhaft verhindert, dass der Ringsteg an das proximale Ende des Produktbehälters 14 anstößt oder zumindest in dem Produktbehälter 14 hineinbewegt wird.

In der ersten und der zweiten Ausführungsform (Figuren 1 und 2) stützt sich die erste Feder 11 mit ihrem proximalen Ende an dem von den Gehäuse 4 gebildeten und nach innen ragenden Ringsteg ab, welcher das Widerlager 4a bildet. Die Feder 11 stützt sich mit ihrem distalen Ende an einem insbesondere ringförmigen, das Vortriebsglied 8 vorzugsweise umgebenden Zwischenglied 11c ab. Die zweite Feder 11b stützt sich mit ihrem distalen Ende an einem Ringsteg oder ringförmigen Kragen, zum Beispiel im Bereich des distalen Endes 8d des Vortriebsglieds 8 ab. Die zweite Feder 11b stützt sich mit ihrem proximalen Ende an dem Zwischenglied 11c ab. Das Zwischenglied 11c koppelt das Vortriebsglied 8 und das Gehäuse 4 so, dass das Vortriebsglied 8 relativ zu dem Gehäuse 4 entlang der Längsachse L verschiebbar und um die Längsachse L verdrehgesichert ist. Das Zwischenglied 11c und die Innenhülse 4a des Gehäuses 4 greifen so ineinander, dass das Zwischenglied 11c drehfest und axial verschiebbar in Bezug auf das Gehäuse 4 ist. Das Zwischenglied 11c und das Vortriebsglied 8 greifen so ineinander, dass das Zwischenglied 11c relativ zu dem Vortriebsglied 8 entlang der Längsachse L verschiebbar und um die Längsachse L drehfest ist.

Die zweite Feder 11b weist einen Außendurchmesser auf, der kleiner ist als der Außendurchmesser der ersten Feder 11a. Hierdurch kann sichergestellt werden, dass der Bereich des Vortriebsglieds 8 bzw. der ersten Feder 11b, welcher für die Produktausschüttung in dem Produktbehälter 14 verschoben wird, ausreichend Abstand zu der den Kolben 14a führenden Innenwand des Produktbehälters 14 aufweist. Das proximale Ende der zweiten Feder 11b ist distal des distalen Endes der ersten Feder 11a angeordnet.

Das Zwischenglied 11c ist so angeordnet, dass es entlang der Längsachse L von einem proximalen Ende des Produktbehälters 14 beabstandet ist, wenn die aus dem Produktbehälter 14 maximal ausschüttbare Produktmenge ausgeschüttet ist, d. h. bei im Wesentlichen vollständig entleerten Produktbehälter 14. Hierdurch wird sichergestellt, dass die erste und die zweite Feder 11a, 11b bis zur vollständigen Produktausschüttung am Ausschüttprozess beteiligt sind.

In der ersten Ausführungsform umgibt die erste Feder 11a das Vortriebsglied 8, wobei die zweite Feder 11b das Vortriebsglied 8 umgibt. Mit anderen Worten sind die erste Feder 11a und die zweite Feder 11b in dem zwischen den Vortriebsglied 8 und der Innenhülse 4a des Gehäuses 4 gebildeten Ringspalt angeordnet. Diese Anordnung erlaubt eine besonders einfache Montage der Antriebs- und Dosiervorrichtung.

In der zweiten Ausführungsform (Figur 2) umgibt die erste Feder 11a das Vortriebsglied 8, wobei das Vortriebsglied 8 die zweite Feder 11b umgibt. Die erste Feder 11a ist anders ausgedrückt in dem Ringspalt zwischen der Innenhülse 4a und dem Vortriebsglied 8 angeordnet. Die zweite Feder 11b ist in dem zwischen dem Rotationsglied 1 und dem Vortriebsglied 8 gebildeten Ringspalt angeordnet. Das hülsenförmige Vortriebsglied 8 weist mindestens einen länglichen, sich parallel zur Längsachse L erstreckenden Schlitz 8f auf, durch den das Zwischenglied 11c greift und in dem das Zwischenglied 11c verschiebbar ist. Die erste Feder 11a und die zweite Feder 11b stützen sich an dem Zwischenglied 11c ab. Der Schlitz 8f kann vorteilhaft für den drehfest und axial verschiebbaren Eingriff des Zwischenglieds 11c mit dem Vortriebsglied 8 dienen.

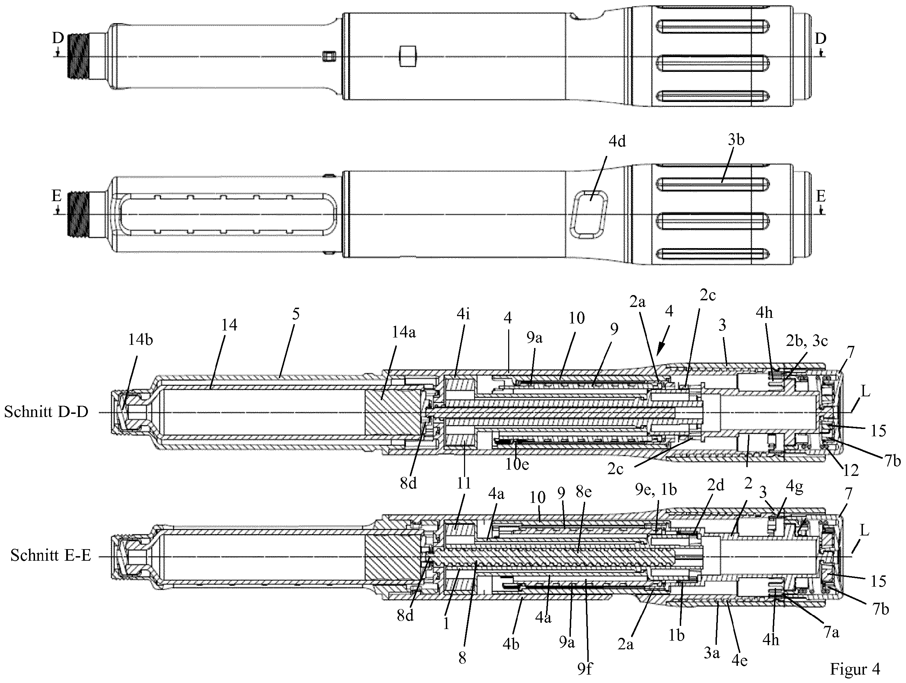

In der in Figur 4 gezeigten vierten Ausführungsform, die keinen Teil der Erfindung formt, ist das Rotationsglied 1 hülsenförmig und umgibt das länglich, insbesondere als Gewindestange ausgebildete Vortriebsglied 8. Das Vortriebsglied 8 und das Rotationsglied 1 greifen so ineinander, dass das Vortriebsglieds 8 in Bezug auf das Rotationsglied 1 um die Längsachse L drehfest und entlang der Längsachse L verschiebbar ist. Das Vortriebsglied 8 weist ein Außengewinde auf, welches in einem Gewindeeingriff mit einem Innengewinde des Gehäuses 4 ist. Eine Drehung des Rotationsglieds 1 relativ zu dem Gehäuse 4 bewirkt eine Drehung des Vortriebsglieds 8, wodurch sich das Vortriebsglied 8 an dem Gehäuse 4 entlang der Längsachse L schraubt.

Die Feder 11 ist in der vierten Ausführungsform eine aus einem bandförmigen Material spiralförmig gewickelte Feder, die als Torsions- oder Drehfeder wirkt. Solche Federn werden auch als Uhrenfedern bezeichnet.

Die Feder 11 stützt sich mit einem Ende an dem Rotationsglied 1 und dem anderen Ende an dem Gehäuse 4 oder dem gehäusefesten Gehäuseeinsatz, der die Innenhülse 4a bildet, ab. Die Innenhülse 4a ist über einen Ringsteg mit dem Gehäuse 4 bzw. der Außenhülse 4b verbunden, wobei die Feder 11 distal des Ringstegs angeordnet ist. Die Feder 11 ist insbesondere distal eines Dosisanzeigeelements 10 und/oder eines Lagerelements 9 angeordnet. Das Lagerelement 9 oder/und das Dosisanzeigeelement 10 sind proximal des Ringstegs angeordnet. Die Feder 11 ist zwischen dem Ringsteg, der die Innenhülse 4a mit der Außenhülse 4b verbindet und dem Ringsteg, welcher das Innengewinde für den Gewindeeingriff mit dem Vortriebsglied 8 aufwiest, angeordnet. Diese Anordnung erlaubt besonders vorteilhaft, dass das von der Feder 11 erzeugte Drehmoment auf möglichst kurzem Weg während der Produktausschüttung in das Vortriebsglied 8 geleitet wird.

Besonders bevorzugt ist der Außendurchmesser der Feder 11 größer als der Innendurchmesser des hülsenförmigen Abschnitts 4a.

Es wird wieder allgemein auf die erste bis vierte Ausführungsform Bezug genommen. Durch Drehung des Rotationsglieds 1 relativ zu dem Gehäuse 4 und dem Vortriebsglied 8 kann die Feder 11 das Vortriebsglied 8 um einen Ausschütthub in distale Richtung verschieben, der proportional zu dem Drehwinkel des Rotationsglieds 1 ist. Durch wahlweises Sperren und Freigeben des Rotationsglieds 1, was durch Betätigen eines als Betätigungsknopf ausgestalteten Betätigungsglieds 7 bewirkt werden kann, kann die Bewegung des Vortriebsglieds 8 relativ zu dem Gehäuse 4, d. h. der Ausschütthub des Vortriebsglieds 8 auf eine vorteilhafte Weise gesteuert werden.

Die Antriebs- und Dosiervorrichtung weist ferner ein Lagerelement 9 auf, welches auch als Anzeigetrommellagerelement bezeichnet werden kann und relativ zu dem Gehäuse 4 drehfest aber entlang der Längsachse L verschiebbar angeordnet ist. Das Lagerelement 9 ist hülsenförmig und umgibt vorzugsweise die Innenhülse 4a des Gehäuses 4, wobei insbesondere die Außenhülse 4b das Lagerelement 9 umgibt. Das Lagerelement 9 ist in einem Eingriff mit dem Gehäuse 4, insbesondere der Innenhülse 4a, der eine Längsbewegung des Lagerelements 9 relativ zu dem Gehäuse 4 zulässt, aber eine Drehbewegung verhindert. Der Eingriff kann durch eine Längsführung 9f zwischen dem Lagerelement 9 und der Innenhülse 4a gebildet werden.

Das Lagerelement 9 weist ein Gewinde 9a, insbesondere ein Außengewinde auf, in welches ein Gewinde 10e, insbesondere ein Innengewinde, des Dosisanzeigeelements 10 eingreift. Durch diesen Gewindeeingriff ist das Anzeigeelement 10 relativ zu dem Lagerelement 9 schraubbar.

Die gezeigten Ausführungsformen umfassen einen Signalerzeugungsmechanismus für die Dosiseinstellung (Dosierklickeinrichtung) und einen Signalerzeugungsmechanismus für die Produktausschüttung (Ausschüttklickeinrichtung), die jeweils ein akustisches und/oder taktiles Signal bei der Dosiseinstellung bzw. bei der Produktausschüttung erzeugen.

Die Ausschüttklickeinrichtung weist einen ringförmigen Klicker 15 auf, der in dem Betätigungsglied 7 angeordnet ist und ein Rastglied aufweist, welches in eine über den Umfang sich erstreckende Innenverzahnung 7b des Betätigungsglieds 7 federnd oder elastisch nachgiebig eingreift.. Der Klicker 15 und das Kupplungsglied 2 sind in einem um die Längsachse L verdrehgesicherten Eingriff, so dass der Klicker 15 von dem Kupplungsglied 2 mitgedreht wird, wenn das Kupplungsglied 2 relativ zu dem Gehäuse 4 um die Längsachse L verdreht wird. Eine Drehung des hülsenförmigen Kupplungsglieds 2 relativ zu dem Gehäuse 4 und/oder dem Betätigungsglied 7 bewirkt, dass das Rastglied des Klickers 15 über die Innenverzahnung 7b rastet und dabei das akustische und/oder taktile Signal erzeugt.

Die Dosierklickeinrichtung weist ein an dem Kupplungsglied 2 federnd gebildetes Eingriffsglied auf, welches in die Außenverzahnung, welche die zweite Kupplungsstruktur 1b bildet, eingreift. Das Kupplungsglied 2 dreht sich während der Dosiseinstellung relativ zu dem Rotationsglied 1, wodurch das Rastglied 2c über die zweite Kupplungsstruktur 1b rastet und dabei das akustische und/oder taktile Signal während der Dosiseinstellung erzeugt. Die Außenverzahnung ist so gestaltet, insbesondere der Abstand zwischen den Zähnen, dass sie die Einstellung von diskreten dosisproportionalen Winkelschritten und/oder die Erzeugung eines leichten Widerstands bei der Dosiseinstellung und/oder die Erzeugung des akustischen oder taktilen Signals, wie zum Beispiel eines hör- und fühlbaren Klicks bei der Dosiseinstellung, ermöglicht

Das Dosisanzeigeelement 10 ist drehfest, aber axial verschiebbar mit dem Kupplungsglied 2 verbunden, insbesondere in einem Eingriff. Dieser Eingriff umfasst eine Längsführung 2a, welche bewirkt, dass das Dosisanzeigeelement 10 relativ zu dem Kupplungsglied 2 drehfest, aber axial verschiebbar ist. Eine Drehung des Kupplungsglieds 2 relativ zu dem Lagerelement 9 bewirkt aufgrund der drehfesten Verbindung zwischen Kupplungsglied 2 und Dosisanzeigeelement 10, dass das Dosisanzeigeelement 10 ebenfalls gedreht und aufgrund des Gewindeeingriffs in das Gewinde 9a an dem Lagerelement 9 entlang geschraubt wird, insbesondere zusätzlich zu dem aufgrund der Rastglieder erzeugten Klickgeräusche.

Das Dosisanzeigeelement 10 weist über seinen Außenumfang eine sich entsprechend der Steigung des Gewindes 10e wendelförmig ersteckende Dosisskala auf, die eine Vielzahl nacheinander angeordnete Skalenwerte umfasst. In dem gezeigten Beispiel kann mit der Antriebs- und Dosiervorrichtung eine maximale Dosis von 80 I.U. eingestellt werden, wobei die Skala von 0 bis 80 reicht und die Dosiswerte in Zweierschritten angegeben sind.

Das Dosisanzeigeelement 10 weist an seinem z.B. proximalen Ende eine in Umfangsrichtung weisende und wirkende Anschlagfläche auf, die als Nulldosisanschlag bezeichnet wird. Das Dosisanzeigeelement 10 weist an seinem, z.B. dem proximalen Ende entgegengesetzten, distalen Ende eine in Umfangsrichtung weisende und wirkende Anschlagfläche auf, welche als Maximaldosisanschlag bezeichnet wird.

Das Dosisanzeigelement 10 ist an dem Lagerelement 9 zwischen einer Nulldosisposition und einer Maximaldosisposition hin und her schraubbar. In der Nulldosisposition verhindert der Nulldosisanschlag im Zusammenwirken mit einem von dem Gehäuse 4 gebildeten Nulldosisgegenanschlag die Drehung des Dosisanzeigeelements 10 in eine erste Drehrichtung, nämlich eine Drehrichtung, die bewirken würde, dass eine kleinere Dosis als Null eingestellt wird. In dieser Nulldosisposition ist das Dosisanzeigeelement 10 in die entgegengesetzte, d. h. zweite Drehrichtung drehbar.

In der Maximaldosisposition verhindert der Maximaldosisanschlag im Zusammenwirken mit einem Maximaldosisgegenanschlag, der von dem Lagerelement 9 gebildet wird, die Drehung des Dosisanzeigeelements 10 in die zweite Drehrichtung, welche eine Erhöhung der Dosis über den maximal einstellbaren Wert hinaus bewirken würde. Die Drehung in die erste Drehrichtung ist in der Maximaldosisposition möglich. Obgleich der Maximaldosisgegenanschlag von dem Lagerelement 9 gebildet wird, kann abweichend von diesem Beispiel der Maximaldosisgegenanschlag optional von dem Gehäuse 4 gebildet werden. Der Nulldosisgegenanschlag kann abweichend von dem gezeigten Beispiel von dem Lagerelement 9 gebildet werden.

Das Gehäuse 4 weist eine Zeigeeinrichtung 4d in der Gestalt eines Fensters auf, welches den Blick auf die Skala des Dosisanzeigeelements 10 frei gibt. An dem Gehäuse 4 ist ein Dosierglied 3 in der Gestalt eines Dosierknopfs drehbar aber axial fest gelagert. Hierfür weist das Gehäuse 4 eine Ringnut 4g auf, in welche insbesondere eine Ringschulter des Dosierglieds 3 eingreift. Das Dosierglied 3 weist über seinen Außenumfang eine Griffstruktur 3b auf, welche es dem Verwender der Vorrichtung erleichtert, das Dosierglied 3 relativ zu dem Gehäuse 4 zu verdrehen. Im unbetätigten Zustand der Vorrichtung bewirkt eine Drehung des Dosierglieds 3 eine Drehung oder Schraubbewegung des Dosisanzeigeelements 10, wodurch die gewünschte Dosis einstellbar und in der Zeigeeinrichtung 4d ablesbar ist.

An dem Dosierglied 3 ist ein Betätigungsglied 7 in der Gestalt eines Betätigungsknopfs angeordnet, welches relativ zu dem Dosierglied 3 für eine Betätigung der Vorrichtung zur Produktausschüttung bewegbar ist, insbesondere entlang der Längsachse L. Das Betätigungsglied 7 bildet das proximale Ende der Vorrichtung und ist vom Daumen der Hand des Verwenders, welche das Gehäuse 4 umgreift, auf einfache Weise betätigbar, insbesondere relativ zu dem Gehäuse 4 und/oder zu dem Dosierglied 3 verschiebbar. Das Kupplungsglied 2 ist relativ zu dem Betätigungsglied 7, insbesondere bei gelöster Dosierkupplung 2b, 3c, drehbar und axial fest. Vorzugsweise ist das Betätigungsglied 7 mit dem Kupplungsglied 2 axial fest aber drehbar verschnappt.

Die Antriebs- und Dosiervorrichtung weist ferner eine Rücksetz- oder Kupplungsfeder 12 auf, die beim Betätigen, insbesondere Drücken des Betätigungsglieds 7 gespannt wird und die das Lagerelement 9 und/oder das Betätigungsglied 7 in seine unbetätigte Position zurücksetzt, wenn das Betätigungsglied 7 unbetätigt ist. Eine Betätigung des Betätigungsglieds 7 bewirkt neben seiner axialen Verschiebung ebenfalls die axiale Verschiebung des Lagerelements 9 entlang der Längsachse L. Die Feder 12 stützt sich mit ihrem distalen Ende vorzugsweise an dem Dosierglied 3 und mit ihrem proximalen Ende vorzugsweise an dem Betätigungsglied 7 ab. Die Feder 12 ist vorzugsweise eine Schrauben- oder Wendelfeder, welche als Druckfeder wirkt.

Das Dosierglied 3 ist relativ zu dem Betätigungsglied 7 drehfest. Das Betätigungsglied 7 durchgreift eine nach innen ragende Schulter des Dosierglieds 3. An dem distalen Ende des vorzugsweise topfförmig ausgestalteten Betätigungsglieds 7 ist eine achte Kupplungsstruktur 7a in Form einer Außenverzahnung gebildet, welche durch Betätigung des Betätigungsglieds 7 mit einer an dem Gehäuse 4 gebildeten siebten Kupplungsstruktur 4h in Form einer Innenverzahnung, insbesondere am proximalen Ende des Gehäuses 4, in einen Eingriff gekuppelt wird, wodurch das Dosierglied 3 in Bezug auf das Gehäuse 4 drehfest ist. Dies bewirkt, dass bei betätigter Vorrichtung eine Dosiseinstellung, d. h. eine Verdrehung des Dosierglieds 3 relativ zu dem Gehäuse 4 nicht möglich ist, sondern erst dann, wenn das Betätigungsglied 7 unbetätigt ist. Die siebte und achte Kupplungsstruktur 4h, 7a bilden eine vierte Kupplung 4h, 7a.

Zwischen dem Dosierglied 3 und dem Kupplungsglied 2 ist eine dritte Kupplung 2b, 3c gebildet, Das Dosierglied 3 bildet eine sechste Kupplungsstruktur 3c der dritten Kupplung 2b, 3c, insbesondere an der nach innen ragenden Schulter. Die sechste Kupplungsstruktur 3c ist bei unbetätigtem Betätigungsglied 7 mit einer als Außenverzahnung am Kupplungsglied 2 gebildet fünften Kupplungsstruktur 2b der dritten Kupplung 2b, 3c drehfest eingekuppelt. Im unbetätigten Zustand des Betätigungsglieds 7 ist die dritte Kupplung 2b, 3c eingekuppelt. Die dritte Kupplung 2b, 3c kann auch als Dosierkupplung bezeichnet werden, die bei der Dosiseinstellung, d. h. bei unbetätigtem Betätigungsglied 7 eingekuppelt und bei der Dosisausschüttung, d. h. bei betätigtem Betätigungsglied 7 ausgekuppelt ist, wobei die dritte Kupplung 2b, 3 im eingekuppelten Zustand Drehmoment überträgt und im ausgekuppelten Zustand kein Drehmoment überträgt. Die dritte Kupplung 2b, 3c wird durch eine Verschiebung des Kupplungsglieds 2 relativ zu dem Gehäuse 4 ausgekuppelt, insbesondere aufgrund der Betätigung des Betätigungsglieds 7.

Das Lagerelement 9 weist an seinem proximalen Ende am Innenumfang eine erste Kupplungsstruktur 9e auf, welche durch eine über den Umfang angeordnete Innenverzahnung gebildet wird, die mit der die zweite Kupplungsstruktur 1b bildenden Verzahnung des Rotationsglieds 1 in einem Eingriff sind, insbesondere bei unbetätigtem Betätigungsglied 7. Die erste Kupplungsstruktur 9e und die zweite Kupplungsstruktur 1b bilden eine erste Kupplung 1b, 9e. Wenn die Kupplungsstruktur 9e, 1b ineinandergreifen, d. h. die Kupplung 1b, 9e eingekuppelt ist, ist das Rotationsglied 1 in Bezug auf das Gehäuse 4 drehfest. Das Kupplungsglied 2 weist ferner eine dritte Kupplungsstruktur 2d an einem Innenumfang auf, die eine über den Umfang angeordnete Innenverzahnung aufweist. Die dritte Kupplungsstruktur 2d ist so angeordnet, dass sie bei Betätigung des Betätigungsglieds 7 in einen drehfesten Eingriff mit dem Rotationsglied 1 gelangt, insbesondere mit der zweiten Kupplungsstruktur 1b oder alternativ einer von der zweiten Kupplungsstruktur 1b separaten, in diesen Beispielen jedoch nicht gezeigten, vierten Kupplungsstruktur. Die dritte Kupplungsstruktur 2b und zweite oder vierte Kupplungsstruktur 1b bilden eine zweite Kupplung 1b, 2d.

Während das Betätigungsglied 7 für die Betätigung relativ zu dem Dosierglied 3 entlang der Längsachse L verschoben wird, wird zusätzlich die zweite Kupplung 1b, 2d eingekuppelt. Durch weiteres Verschieben des Betätigungsglieds 7 relativ zu dem Dosierglied 3 wird die erste Kupplung 1b, 9e eingekuppelt. Bevor, nachdem oder gleichzeitig mit dem Auskuppeln der ersten Kupplung 1b, 9e wird die dritte Kupplung 2b, 3c ausgekuppelt. und/oder die vierte Kupplung 4h, 7a eingekuppelt.

Insbesondere dadurch, dass die erste Kupplungsstruktur 9e von der zweiten Kupplungsstruktur 1b gelöst ist, kann die mindestens eine Ausschüttfeder 11; 11a, 11b sich entspannen, wobei das Rotationsglied 1 relativ zu dem Gehäuse 4 verdreht wird und aufgrund des Eingriffs der zweiten Kupplungsstruktur 1b mit der dritten Kupplungsstruktur 2d das Kupplungsglied 2 und somit auch das Dosisanzeigeelement 10 relativ zu dem Gehäuse 4 verdreht werden, wodurch das Dosisanzeigeelement 10 in seine Nulldosisposition zurück geschraubt und das Vortriebsglied 8 proportional zu dem sich insbesondere in Umfangsrichtung erstreckenden Abstand zwischen Nulldosisanschlag 10c und Nulldosisgegenanschlag 4f um einen Ausschütthub relativ zu dem Gehäuse 4 in distale Richtung verschoben wird. Die Drehung des Kupplungsglieds 2 relativ zu dem Betätigungsglied 7 bewirkt, dass die Rastglieder des Klickers 15 über die Verzahnung 7b insbesondere in dosisproportionalen Winkelschritten rasten und dabei das akustische und /oder taktile Signal erzeugen.

Die Antriebs- und Dosiervorrichtung weist einen Dosisbegrenzer in der Gestalt eines Rings, eines Ringsegments oder einer Mutter auf, der an seinem Innenumfang ein Gewinde aufweist, das in ein an einem Außenumfang des Gehäuses 4 angeordnetes Gewinde 4e eingreift, so dass der Dosisbegrenzer relativ zu dem Gehäuse 4 schraubbar ist. Am Außenumfang weist der Dosisbegrenzer ein Eingriffsglied auf, welches in eine Längsführung 3a am Innenumfang des Dosierglieds 3 eingreift, so dass das der Dosisbegrenzer relativ zu dem Dosierglied 3 drehfest aber axial verschiebbar ist. An dem Dosierglied 3 oder dem Gehäuse 4 ist ein Stoppanschlag gebildet, von dem der Dosisbegrenzer proportional zu der maximal aus dem Produktbehälter 14 ausschüttbaren Produktmenge beabstandet ist. Da bei der Dosiseinstellung das Dosierglied 3 relativ zu dem Gehäuse 4 verdreht und bei einer Dosisausschüttung nicht verdreht wird, kann durch den Dosisbegrenzer ein Zählwerk gebildet werden, welches die bereits ausgeschütteten Einzeldosen und die aktuell eingestellte Dosis addiert und sich dementsprechend immer näher an den Stoppanschlag des Dosierglieds 3 oder des Gehäuses 4 annähert. Eine Dosiserhöhung bewirkt, dass der Dosisbegrenzer zu dem Stoppanschlag hin bewegt wird. Eine Dosisverringerung bewirkt, dass der Dosisbegrenzer von dem Stoppanschlag weg bewegt wird. Ist die in dem Produktbehälter 14 angegebene Restdosis geringer als die maximal mit der Antriebs- und Dosiervorrichtung einstellbare Dosis, gerät der Dosisbegrenzer in einen Kontakt mit dem Stoppanschlag, so dass eine Verdrehung des Dosierglieds 3 relativ zu dem Gehäuse 4 in eine Drehrichtung, die eine Erhöhung der Dosis zur Folge hätte, blockiert wird.

Das System aus erster Kupplung 1b, 9e und zweiter Kupplung 1b, 2d kann auch als Ausschüttkupplung bezeichnet werden.

In den Figuren 1 bis 3 wird die Antriebs- und Dosiervorrichtung, die auch als Injektionsvorrichtung bezeichnet werden kann, in ihrem Ausgangs- oder Auslieferungszustand, insbesondere vor einer ersten Verwendung gezeigt. Die in der Zeigeeinrichtung 4d angezeigte Produktdosis ist 0. Ein Betätigen des Betätigungsglieds 7 hätte zur Folge, dass keine Dosis ausgeschüttet wird. Der Dosisbegrenzer weist einen Abstand zu dem Stoppanschlag auf, welcher proportional zu der in dem Produktbehälter 14 enthaltenen oder ausschüttbaren Produktmenge ist, wie z. B. 300 I.U.

Zur Einstellung der Produktdosis wird das Dosiseinstellglied 3 relativ zu dem Gehäuse 4 verdreht, wodurch aufgrund der eingekuppelten dritten Kupplung 2b, 3c das Kupplungsglied 2 und somit auch das Dosisanzeigeelement 10 relativ zu dem Gehäuse 4 verdreht werden. Dabei schraubt sich das Dosisanzeigeelement 10 entlang dem Lagerelement 9 aufgrund des Gewindeeingriffs des Gewindes 10e mit dem Gewinde 9a. Insbesondere wird der Abstand zwischen dem Nulldosisanschlag und dem Nulldosisgegenanschlag proportional zu der in der Zeigeeinrichtung 4d gezeigten Dosis erhöht. Außerdem wird bei der Drehung aufgrund des Überrastens der Rastglieder über die Verzahnung 1b ein hörbares und fühlbares Signal erzeugt.

Wenn eine maximal einstellbare Dosis eingestellt wurde, wie zum Beispiel 80 I.U., die dann in der Zeigeeinrichtung 4d ablesbar sind, ist eine weitere Dosiserhöhung ist aufgrund des Zusammenwirkens, insbesondere des Kontakts des Maximaldosisanschlags mit dem Maximaldosisgegenanschlag nicht möglich. Der Dosisbegrenzer ist entsprechend 80 I.U. weit an den Stoppanschlag herangerückt oder verschoben.

Zur Ausschüttung der eingestellten bzw. angezeigten Dosis wird das Betätigungsglied 7 betätigt, insbesondere gedrückt, d. h. relativ zu dem Gehäuse 4 und dem Dosierglied 3 in distale Richtung verschoben, wodurch das Kupplungsglied 2 und das Lagerelement 9 sowie das Dosisanzeigeelement 10 relativ zu dem Gehäuse 4 in distale Richtung verschoben werden, insbesondere gegen die Kraft der Kupplungs- oder Rücksetzfeder 12.

Die Betätigung des Betätigungsglieds 7 bewirkt, dass die zweite Kupplung 1b, 2d einkuppelt und die erste Kupplung 1b, 9e auskuppelt, so dass das Rotationsglied 1 in Bezug auf das Gehäuse 4 nicht mehr drehfest sondern drehbar ist und in Bezug auf das Kupplungsglied 2 und das Dosisanzeigeelement 10 drehfest ist. Die Betätigung des Betätigungsglieds 7 bewirkt ferner, dass die dritte Kupplung 2b, 3c ausgekuppelt wird und die vierte Kupplung 4h, 7a eingekuppelt wird. Im betätigten Zustand des Betätigungsglieds 7 ist das Rotationsglied 1 relativ zu dem Dosisanzeigeelement 10 drehfest, wodurch sich das Rotationsglieds 1 und das Dosisanzeigeelement 10 gemeinsam relativ zu dem Gehäuse 4 drehen können. Durch die Kraft der in der mindestens einen Ausschüttfeder 11; 11a, 11b gespeicherten Energie auf das Vortriebsglied 8 wird aufgrund des Gewindeeingriffs des Vortriebsglieds 8 mit dem Rotationsglied 1 eine Drehung des Rotationsglieds 1 und des Dosisanzeigeelements 10 relativ zu dem Gehäuse 4 bewirkt, wodurch sich das Dosisanzeigeelement 10 an dem Lagerelement 9 in Richtung Nulldosisposition zurückschraubt und wobei die in der Zeigeeinrichtung 14d angezeigte Dosis runterzählt. Gleichzeitig wird das Vortriebsglied 8 von der mindestens einen Ausschüttfeder 11; 11a, 11b relativ zu dem Gehäuse 4 in distale Richtung um den Ausschütthub bewegt, welcher proportional zu der zuvor eingestellten Dosis ist. Wenn das Dosisanzeigeelement 10 seine Nulldosisposition erreicht hat ist die zuvor eingestellte Dosis oder Einzeldosis ausgeschüttet. Lässt der Verwender das Betätigungsglied 7 los, setzt die Kupplungs- oder Rücksetzfeder 12 das Betätigungsglied 7, das Kupplungsglied 2, das Lagerelement 9 und das Dosisanzeigeelement 10 zurück. Beim Rücksetzen werden die genannten Elemente relativ zu dem Gehäuse 4 oder dem Dosierglied 3 in proximale Richtung verschoben.

Beim Zurücksetzen der Vorrichtung mittels der Feder 12 wird die erste Kupplung 1b, 9e eingekuppelt und die zweite Kupplung 1b, 2d auskuppelt. Das Rotationsglied 1 ist nun wieder drehfest in Bezug auf das Gehäuse 4, wobei das Dosierglied 3 zusammen mit dem Dosisanzeigeelement 10 relativ zu dem Gehäuse 4 und/oder der Zeigeeinrichtung 4d und/oder dem Rotationsglied 1 für eine neue Einstellung einer Produktdosis oder Einzeldosis drehbar ist. Beim Zurücksetzen werden ferner die vierte Kupplung 4h, 7a ausgekuppelt und die dritte Kupplung 2b, 3c eingekuppelt, wodurch das Dosierglied 3 relativ zu dem Kupplungsglied 2 und dem Dosisanzeigeelement 10 drehfest ist.

Beispielhaft sei angenommen, nach mehreren Verabreichungen sind noch 76 I.U. in dem Produktbehälter 14 enthalten. Mit der Antriebs- und Dosiervorrichtung wären maximal 80 I.U. einstellbar. Da der Dosisbegrenzer bereits bei 76 I.U. in einem Kontakt mit dem Stoppanschlag ist, wird das Dosierglied 3 für eine Drehung in die zweite Richtung, welche eine Erhöhung der Dosis bewirken würde, gesperrt. Die Verringerung der Dosis ist jedoch durch Drehen des Dosierglieds 3 in die erste Drehrichtung möglich.

Durch Betätigen des Betätigungsglieds 7 wird die in der Zeigeeinrichtung 4d gezeigte Dosis ausgeschüttet. Da anschließend der Produktbehälter 14 vollständig entleert ist, wird die Antriebs- und Dosiervorrichtung oder Injektionsvorrichtung als Ganzes entsorgt. Es handelt sich somit um eine Einweg-Injektionsvorrichtung. Grundsätzlich können die hierin gezeigten Antriebs- und Dosiervorrichtungen aber auch in Verbindung mit Mehrweginjektionsvorrichtungen Anwendung finden, bei denen ein entleerter Produktbehälter 14 gegen einen neuen ausgetauscht wird.

**Bezugszeichenliste**

| | | | |
|---|---|---|---|
| 1 | Rotationsglied / Gewindestange / Gewindemutter | 8d | distales Ende |
| | | 8e | Gewindestange |
| 1a | Außengewinde | 8f | Schlitz |
| 1b | zweite und/oder vierte Kupplungsstruktur / Verzahnung | 8g | Schulter / Ringsteg |
| | | 9 | Lagerelement |
| 2 | Kupplungsglied / Anzeigekupplung | 9a | Außengewinde |
| 2a | Längsführung | 9e | erste Kupplungsstruktur / Zähne |
| 2b | fünfte Kupplungsstruktur | 9f | Längsführung |
| 2c | Rastglied | | |
| 2d | dritte Kupplungsstruktur | 10 | Dosisanzeigeelement / Dosisanzeigetrommel |
| 3 | Dosierglied / Dosierknopf / Dosiseinstellglied | 10e | Innengewinde |
| 3a | Längsführung | 11 | Feder / Ausschüttfeder / Druckfeder / Torsionsfeder |
| 3b | Griffstruktur | | |
| 3c | sechste Kupplungsstruktur | 11a | erste Ausschüttfeder |
| | | 11b | zweite Ausschüttfeder |
| 4 | Gehäuse | 11c | Zwischenglied |
| 4a | Innenhülse | | |
| 4b | Außenhülse | 12 | Feder / Rücksetz- oder Kupplungsfeder |
| 4c | Längsführung | | |
| 4d | Zeigeeinrichtung / Fenster | | |
| 4e | Gewinde / Außengewinde | 14 | Produktbehälter / Karpule |
| 4g | Ringnut | 14a | Kolben |
| 4h | siebte Kupplungsstruktur / Innenverzahnung 4i Widerlager | 14b | Septum |
| | | 15 | Klicker |
| 5 | Produktbehälteraufnahme | | |
| | | 1b, 9e | erste Kupplung |
| 7 | Betätigungsglied / Betätigungsknopf | 1b, 2d | zweite Kupplung |
| 7a | achte Kupplungsstruktur / Außenverzahnung | 2b, 3c | dritte Kupplung |
| | | 4h, 7a | vierte Kupplung |
| 7b | Innenverzahnung | | |
| | | | |
| 8 | Vortriebsglied / Stößel | | |
| 8a | Längsrippe | | |
| 8b | Innenhülse | | |
| 8c | Gewinde / Innengewinde | | |

## Patentansprüche

1. Antriebs- und Dosiervorrichtung für eine Injektionsvorrichtung zur Verabreichung eines flüssigen Produkts, insbesondere eines Medikaments, wobei mit der Antriebs- und Dosiervorrichtung wiederholt zu verabreichende Produktdosen einstellbar sind, umfassend:
a) ein Gehäuse (4) und ein insbesondere vom Verwender der Antriebs- und Dosiervorrichtung greifbares Dosierglied (3), welches zur Einstellung einer zu verabreichenden Produktdosis relativ zu dem Gehäuse (4) drehbar ist,
b) ein insbesondere vom Verwender der Antriebs- und Dosiervorrichtung betätigbares Betätigungsglied (7), das zur Ausschüttung der eingestellten Produktdosis relativ zu dem Dosierglied (3) verschiebbar ist,
c) ein Vortriebsglied (8), dessen distales Ende (8d) vorgesehen ist, auf einen Kolben (14a) eines an der Antriebs- und Dosiervorrichtung befestigbaren Produktbehälters (14) zu wirken,
d) ein Widerlager (4i), wobei das Vortriebsglied (8) relativ zu dem Widerlager (4i) in eine Ausschüttrichtung bewegbar ist, um eine Ausschüttung der eingestellten Produktdosis bewirken zu können,
e) mindestens eine zwischen dem Vortriebsglied (8) und dem Widerlager (4i) wirkende und im Auslieferungszustand der Vorrichtung vorgespannte Feder (11; 11a, 11b), die mit so viel Energie vorgespannt ist, dass sie die aus dem befestigbaren Produktbehälter (14) maximal ausschüttbare Produktmenge in mehreren Einzelausschüttungen ausschütten kann,
f) ein Rotationsglied (1), dessen Drehung relativ zu dem Gehäuse (4) bewirkt, dass die mindestens eine Feder (11; 11a, 11b) das Vortriebsglied (8) in Ausschüttrichtung bewegen kann,
g) und mindestens eine Kupplung (9e, 1b), die durch Betätigung des Betätigungsglieds (7) die Drehung des Rotationsglieds (1) relativ zu dem Gehäuse (4) freigibt und durch Loslassen des Betätigungsglieds (1) die Drehung des Rotationsglieds (1) relativ zu dem Gehäuse (4) blockiert, wobei
h) die mindestens eine Feder (11; 11a, 11b) eine erste wendelförmige Druckfeder (11a) und eine zweite wendelförmige Druckfeder (11b) umfasst, wobei sich die erste Druckfeder (11a) an dem Widerlager (4i) und einem Zwischenglied (11c) und die zweite Druckfeder (11b) an dem Zwischenglied (11c) und dem Vortriebsglied (8) abstützen, **dadurch gekennzeichnet, dass**
i) das Zwischenglied (11c) das Vortriebsglied (8) drehfest mit dem Gehäuse (4) oder dem Widerlager (4i) koppelt.

2. Antriebs- und Dosiervorrichtung nach Anspruch 1, **dadurch gekennzeichnet, dass** das Zwischenglied (11c) mit dem Gehäuse (4) oder dem Widerlager (4i) in so einem Eingriff ist, dass das Zwischenglied (11c) relativ zu dem Gehäuse (4) entlang der Längsachse (L) verschiebbar und um die Längsachse (L) verdrehfest ist.

3. Antriebs- und Dosiervorrichtung nach Anspruch 1 oder 2, **dadurch gekennzeichnet, dass** das Zwischenglied (11c) mit dem Vortriebsglied (8) in so einem Eingriff ist, dass das Zwischenglied (11c) relativ zu dem Vortriebsglied (8) entlang der Längsachse (L) verschiebbar und um die Längsachse (L) verdrehfest ist.

4. Antriebs- und Dosiervorrichtung nach einem der Ansprüche 1 bis 3, **dadurch gekennzeichnet, dass** die erste Druckfeder (11a) das Vortriebsglied (8) umgibt und die zweite Druckfeder (11b) das Vortriebsglied (8) umgibt.

5. Antriebs- und Dosiervorrichtung nach einem der Ansprüche 1 bis 3, **dadurch gekennzeichnet, dass** die erste Druckfeder (11a) das Vortriebsglied (8) umgibt und das Vortriebsglied (8) die zweite Druckfeder (11b) umgibt.

6. Antriebs- und Dosiervorrichtung nach Anspruch 5, **dadurch gekennzeichnet, dass** das Zwischenglied (11c) sich durch einen länglichen und sich entlang der Längsachse (L) erstreckenden Schlitz (11f) in der Wand des hülsenförmigen Vortriebsglieds (8) erstreckt, wobei das Zwischenglied (11c) während der Produktausschüttung in dem Schlitz (11f) verschoben wird.

7. Antriebs- und Dosiervorrichtung nach einem der Ansprüche 1 bis 6, **dadurch gekennzeichnet, dass** die zweite Druckfeder (11b) distal der ersten Druckfeder (11a) angeordnet ist, wobei die zweite Druckfeder (11b) einen geringeren Außendurchmesser aufweist als die erste Druckfeder (11a), wobei bevorzugt ist, dass die Antriebs- und Dosiervorrichtung einen Produktbehälter (14), der einen an einer Innenwand des Produktbehälters (14) verschiebbar anliegenden Kolben (14a) aufweist, umfasst, wobei zumindest die zweite Druckfeder (11b) einen geringeren Außendurchmesser aufweist als die Innenwand.

8. Antriebs- und Dosiervorrichtung nach einem der Ansprüche 1 bis 6, **dadurch gekennzeichnet, dass** sie einen Produktbehälter (14), der einen verschiebbaren Kolben (14a) aufweist, umfasst und dass das Vortriebsglied (8) so angeordnet ist, dass es auf den Kolben (14a) wirkt, wobei das Zwischenglied (11c) entlang der Längsachse (L) von einem proximalen Ende des Produktbehälters (14) beabstandet ist oder das proximale Ende des Produktbehälters (14) berührt, wenn die aus dem Produktbehälter (14) maximal ausschüttbare Produktmenge ausgeschüttet ist.

9. Antriebs- und Dosiervorrichtung nach einem der vorhergehenden Ansprüche, **dadurch gekennzeichnet, dass** das Widerlager (4i) durch das Gehäuse (4) oder ein gehäusefestes Element gebildet wird und/oder dass das Vortriebsglied (8) an dem Gehäuse (4) oder dem gehäusefesten Element geführt ist.

10. Antriebs- und Dosiervorrichtung nach einem der vorhergehenden Ansprüche, ferner umfassend ein Dosisanzeigeelement (10), über dessen Umfang eine Dosisskala (10b) angeordnet ist, und eine Zeigeeinrichtung (4d), wobei zur Einstellung der zu verabreichenden Dosis durch Drehung des Dosierglieds (3) relativ zu der Zeigeeinrichtung (4d) das Dosisanzeigeelement (10) relativ zu der Zeigeeinrichtung (4d) und um eine Drehachse (L) drehbar, insbesondere schraubbar, ist und mittels der Zeigeeinrichtung (4d) ein Wert der Dosisskala (10b) ablesbar ist, welcher der eingestellten Dosis entspricht, wobei bevorzugt ist, dass das Dosisanzeigeelement (10) einen Anschlag (10c) aufweist, der von einem Gegenanschlag (4f) wegbewegt wird, wenn eine Dosiserhöhung vorgenommen wird, und der zu dem Gegenanschlag (4f) hinbewegt wird, wenn eine Dosisverringerung vorgenommen wird oder wenn die Vorrichtung für die Ausschüttung der eingestellten Produktdosis betätigt wird.

11. Antriebs- und Dosiervorrichtung nach einem der vorhergehenden Ansprüche, **dadurch gekennzeichnet, dass** das Dosierglied (3) zumindest bei der Dosiseinstellung rotatorisch von der mindestens einen Feder (11; 11a, 11b) entkoppelt ist, so dass die mindestens eine Feder (11; 11a, 11b) bei der Dosiseinstellung weder gespannt noch entspannt wird.

## Claims

1. A driving and dosing device for an injection device for administering a liquid product, in particular a drug, wherein product dosages to be administered can be set repeatedly using the driving and dosing device, wherein the driving and dosing device comprises:
a) a housing (4) and a dosing member (3) which can be gripped in particular by the user of the driving and dosing device and which can be rotated relative to the housing (4) in order to set a product dosage to be administered;
b) an activating member (7) which can be activated in particular by the user of the driving and dosing device and which can be shifted relative to the dosing member (3) in order to deliver the product dosage set;
c) an advancing member (8), the distal end (8d) of which is designed to act on a piston (14a) of a product container (14) which can be attached to the driving and dosing device;
d) an abutment (4i), wherein the advancing member (8) can be moved relative to the abutment (4i) in a delivering direction, in order to be able to cause the product dosage set to be delivered;
e) at least one spring (11; 11a, 11b) which acts between the advancing member (8) and the abutment (4i) and is biased in the device as delivered, wherein the spring (11; 11a, 11b) is biased with an amount of energy such that it can deliver, in multiple individual deliveries, the maximum product amount which can be delivered from the attachable product container (14);
f) a rotational member (1) which, when rotated relative to the housing (4), enables the at least one spring (11; 11a, 11b) to move the advancing member (8) in the delivering direction;
g) and at least one coupling (9e, 1b) which, when the activating member (7) is activated, allows the rotational member (1) to rotate relative to the housing (4) and, when the activating member (7) is released, blocks the rotational member (1) from rotating relative to the housing (4), wherein
h) the at least one spring (11; 11a, 11b) comprises at least a first helical pressure spring (11a) and a second helical pressure spring (11b), wherein the first pressure spring (11a) is supported on the abutment (4i) and an intermediate member (11c), and the second pressure spring (11b) is supported on the intermediate member (11c) and the advancing member (8), **characterised in that**
i) the intermediate member (11c) couples the advancing member (8), rotationally fixed, to the housing (4) or to the abutment (4i).

2. The driving and dosing device according to Claim 1, **characterised in that** the intermediate member (11c) is in an engagement with the housing (4) or the abutment (4i) such that the intermediate member (11c) can be shifted relative to the housing (4) along the longitudinal axis (L) and is rotationally fixed about the longitudinal axis (L).

3. The driving and dosing device according to Claim 1 or 2, **characterised in that** the intermediate member (11c) is in an engagement with the advancing member (8) such that the intermediate member (11c) can be shifted relative to the advancing member (8) along the longitudinal axis (L) and is rotationally fixed about the longitudinal axis (L).

4. The driving and dosing device according to any one of Claims 1 to 3, **characterised in that** the first pressure spring (11a) surrounds the advancing member (8), and the second pressure spring (11b) surrounds the advancing member (8).

5. The driving and dosing device according to any one of Claims 1 to 3, **characterised in that** the first pressure spring (11a) surrounds the advancing member (8), and the advancing member (8) surrounds the second pressure spring (11b).

6. The driving and dosing device according to Claim 5, **characterised in that** the intermediate member (11c) extends through an elongated slot (11f), extending along the longitudinal axis (L), in the wall of the sleeve-shaped advancing member (8), wherein the intermediate member (11c) is shifted in the slot (11f) while product is delivered.

7. The driving and dosing device according to any one of Claims 1 to 6, **characterised in that** the second pressure spring (11b) is arranged distally with respect to the first pressure spring (11a), wherein the second pressure spring (11b) exhibits a smaller outer diameter than the first pressure spring (11a), wherein the driving and dosing device preferably comprises a product container (14) comprising a piston (14a) which abuts an inner wall of the product container (14) such that it can be shifted, wherein at least the second pressure spring (11b) exhibits a smaller outer diameter than the inner wall.

8. The driving and dosing device according to any one of Claims 1 to 6, **characterised in that** it comprises a product container (14) comprising a piston (14a) which can be shifted, and **in that** the advancing member (8) is arranged such that it acts on the piston (14a), wherein the intermediate member (11c) is spaced from a proximal end of the product container (14) along the longitudinal axis (L) or contacts the proximal end of the product container (14) when the maximum deliverable product amount is delivered from the product container (14).

9. The driving and dosing device according to any one of the preceding claims, **characterised in that** the abutment (4i) is formed by the housing (4) or by an element which is fixed relative to the housing (4) and/or **in that** the advancing member (8) is guided on the housing (4) or on the element which is fixed relative to the housing (4).

10. The driving and dosing device according to any one of the preceding claims, further comprising a dosage indicator element (10) with a dosage scale (10b) arranged over its circumference, and a pointer (4d), wherein the dosage indicator element (10) can be rotated, in particular screwed, relative to the pointer (4d) and about a rotational axis (L) in order to set the dosage to be administered by rotating the dosing member (3) relative to the pointer (4d), and a value on the dosage scale (10b) which corresponds to the dosage set can be read off by means of the pointer (4d), wherein the dosage indicator element (10) preferably comprises a stopper (10c) which moves away from a counter stopper (4f) when the dosage is increased and moves towards the counter stopper (4f) when the dosage is decreased or when the device is activated for delivering the product dosage set.

11. The driving and dosing device according to any one of the preceding claims, **characterised in that** the dosing member (3) is rotationally decoupled from the at least one spring (11; 11a, 11b), at least when a dosage is being set, such that the at least one spring (11; 11a, 11b) is neither tensed nor relaxed when a dosage is being set.

## Revendications

1. Dispositif d'entraînement et de dosage pour un dispositif d'injection servant à administrer un produit liquide, en particulier un médicament, dans lequel des doses de produit à administrer de manière répétée peuvent être réglées avec le dispositif d'entraînement et de dosage, comprenant :
a) un boîtier (4) et un organe de dosage (3) pouvant être saisi en particulier par l'utilisateur du dispositif d'entraînement et de dosage, lequel organe de dosage peut être tourné par rapport au boîtier (4) afin de régler une dose de produit à administrer,
b) un organe d'actionnement (7) pouvant être actionné en particulier par l'utilisateur du dispositif d'entraînement et de dosage, lequel organe d'actionnement peut être coulissé par rapport à l'organe de dosage (3) afin de déverser la dose de produit réglée,
c) un organe de poussée (8), dont l'extrémité distale (8b) est prévue pour agir sur un piston (14a) d'un contenant de produit (14) pouvant être fixé au niveau du dispositif d'entraînement et de dosage,
d) une butée (4i), dans lequel l'organe de poussée (8) peut être déplacé par rapport à la butée (4i) dans une direction de déversement afin de pouvoir entraîner un déversement de la dose de produit réglée,
e) au moins un ressort (11 ; 11a, 11b) agissant entre l'organe de poussée (8) et la butée (4i) et précontraint dans l'état de livraison du dispositif, lequel ressort est précontraint avec tant d'énergie qu'il peut déverser en plusieurs déversements individuels la quantité de produit pouvant être déversée au maximum hors du contenant de produit (14) pouvant être fixé,
f) un organe de rotation (1), dont la rotation par rapport au boîtier (4) a pour effet que l'au moins un ressort (11 ; 11a, 11b) peut déplacer l'organe de poussée (8) dans la direction de déversement,
g) et au moins un couplage (9a, 1b), qui débloque par l'actionnement de l'organe d'actionnement (7) la rotation de l'organe de rotation (1) par rapport au boîtier (4) et qui bloque par la libération de l'organe d'actionnement (1) la rotation de l'organe de rotation (1) par rapport au boîtier (4), dans lequel
h) l'au moins un ressort (11 ; 11a, 11b) comprend un premier ressort de pression (11a) en forme d'hélice et un deuxième ressort de pression (11b) en forme d'hélice, dans lequel le premier ressort de pression (11a) prend appui au niveau de la butée (4i) et d'un organe intermédiaire (11c) et le deuxième ressort de pression (11b), au niveau de l'organe intermédiaire (11c) et de l'organe de poussée (8), **caractérisé en ce que**
i) l'organe intermédiaire (11c) couple l'organe de poussée (8) de manière solidaire en rotation au boîtier (4) ou à la butée (4i).

2. Dispositif d'entraînement et de dosage selon la revendication 1, **caractérisé en ce que** l'organe intermédiaire (11c) est en prise avec le boîtier (4) ou la butée (4i) de telle sorte que l'organe intermédiaire (11c) peut être coulissé par rapport au boîtier (4) le long de l'axe longitudinal (L) et est solidaire en torsion autour de l'axe longitudinal (L).

3. Dispositif d'entraînement et de dosage selon la revendication 1 ou 2, **caractérisé en ce que** l'organe intermédiaire (11c) est en prise avec l'organe de poussée (8) de telle sorte que l'organe intermédiaire (11c) peut être coulissé par rapport à l'organe de poussée (8) le long de l'axe longitudinal (L) et est solidaire en torsion autour de l'axe longitudinal (L).

4. Dispositif d'entraînement et de dosage selon l'une quelconque des revendications 1 à 3, **caractérisé en ce que** le premier ressort de pression (11a) entoure l'organe de poussée (8), et le deuxième ressort de pression (11b) entoure l'organe de poussée (8).

5. Dispositif d'entraînement et de dosage selon l'une quelconque des revendications 1 à 3, **caractérisé en ce que** le premier ressort de pression (11a) entoure l'organe de poussée (8), et l'organe de poussée (8) entoure le deuxième ressort de pression (11b).

6. Dispositif d'entraînement et de dosage selon la revendication 5, **caractérisé en ce que** l'organe intermédiaire (11c) s'étend à travers une entaille (11f) allongée et s'étendant le long de l'axe longitudinal (L) dans la paroi de l'organe de poussée (8) en forme de douille, dans lequel l'organe intermédiaire (11c) est coulissé dans l'entaille (11f) au cours du déversement de produit.

7. Dispositif d'entraînement et de dosage selon l'une quelconque des revendications 1 à 6, **caractérisé en ce que** le deuxième ressort de pression (11b) est disposé distalement au premier ressort de pression (11a), dans lequel le deuxième ressort de pression (11b) présente un diamètre extérieur plus petit que le premier ressort de pression (11a), dans lequel on préfère que le dispositif d'entraînement et de dosage comprenne un contenant de produit (14), qui présente un piston (14a) reposant de manière à pouvoir coulisser au niveau d'une paroi intérieure du contenant de produit (14), dans lequel au moins le deuxième ressort de pression (11b) présente un diamètre extérieur inférieur à la paroi intérieure.

8. Dispositif d'entraînement et de dosage selon l'une quelconque des revendications 1 à 6, **caractérisé en ce qu'**il comprend un contenant de produit (14), qui présente un piston (14a) pouvant être coulissé, et que l'organe de poussée (8) est disposé de telle sorte qu'il agit sur le piston (14a), dans lequel l'organe intermédiaire (11c) est tenu à distance, le long de l'axe longitudinal (L), d'une extrémité proximale du contenant de produit (14) ou touche l'extrémité proximale du contenant de produit (14) quand la quantité de produit pouvant être déversée au maximum hors du contenant de produit (14) est déversée.

9. Dispositif d'entraînement et de dosage selon l'une quelconque des revendications précédentes, **caractérisé en ce que** la butée (4i) est formée par le boîtier (4) ou par un élément solidaire du boîtier, et/ou que l'organe de poussée (8) est guidé au niveau du boîtier (4) ou au niveau de l'élément solidaire du boîtier.

10. Dispositif d'entraînement et de dosage selon l'une quelconque des revendications précédentes, comprenant en outre un élément d'affichage de dose (10), sur la périphérie duquel est disposée une graduation de dose (10b), et un système de pointage (4d), dans lequel l'élément d'affichage de dose (10) peut être tourné, en particulier vissé, par rapport au système de pointage (4d) et autour d'un axe de rotation (L) afin de régler la dose à administrer par la rotation de l'organe de dosage (3) par rapport au système de pointage (4d) et une valeur de la graduation de dose (10b) peut être lue au moyen du système de pointage (4d), laquelle valeur correspond à la dose réglée, dans lequel on préfère que l'élément d'affichage de dose (10) présente une butée (10c), qui est éloignée par déplacement d'une contre-butée (4f) quand une augmentation de dose est entreprise, et qui est rapproché par déplacement de la contre-butée (4f) quand une réduction de dose est entreprise, ou quand le dispositif est actionné en vue du déversement de la dose de produit réglée.

11. Dispositif d'entraînement et de dosage selon l'une quelconque des revendications précédentes, **caractérisé en ce que** l'organe de dosage (3) est découplé au moins lors du réglage de dose par rotation de l'au moins un ressort (11; 11a, 11b) de sorte que l'au moins un ressort (11 ; 11a, 11b) ne soit ni tendu ni détendu lors du réglage de dose.
